# EUROPEAN PATENT APPLICATION

(11) **EP 1 420 354 A2**
(43) Date of publication of application: **19.05.2004**
(21) Application number: 03256582.2
(22) Date of filing: 20.10.2003
(51) Int. Cl.: G06F 19/00

(54) **Medical image radiographing system, method for displaying radiographing order information, portable terminal, display control method and program**

(30) Priority: 31.10.2002 JP 2002317299; 26.03.2003 JP 2003084801
(71) Applicant: KONICA MINOLTA HOLDINGS, INC., Tokyo 100-0005 (JP)
(72) Inventor: Moriyama, Naoto, Hachioji-shi Tokyo 192-8505 (JP); Motoki, Wataru, Hachioji-shi Tokyo 192-8505 (JP); Shiibashi, Takao, Hachioji-shi Tokyo 192-8505 (JP); Umeki, Mamoru, Hachioji-shi Tokyo 192-8505 (JP)
(74) Representative: Rees, Alexander Ellison

(57) **Abstract**

A medical image radiographing system capable of radiographing a medical image at a patient's location efficiently and accurately. The medicalimage radiographing system has: a radiographing apparatus for radiographing a medical image of a patient; an information management apparatus for managing radiographing order information; a control apparatus for obtaining the radiographing order information from the information management apparatus through a communication network; and a portable terminal connectable to the control apparatus; wherein the control apparatus comprises a storage section for storing the radiographing order information and an assigning section for assigning an operator for performing radiography and transmitting the radiographing order information to the portable terminal, and the portable terminal comprises a storage section for storing the radiographing order information and the operator identification information, an authentication section for authenticating the operator and a display section for displaying the radiographing order information when the authentication section authenticates the operator.

## Description

### BACKGROUND OF THE INVENTION

### Field of the Invention

The present invention relates to a medical image radiographing system. The present invention further relates to a method for displaying radiographing order information, a portable terminal, a display control method and a program in the medical image radiographing system.

### Description of Related Art

In a medical field, digitization of medical images produced by radiographing patients has been achieved, and a medical image radiographing system has been used, the medical image radiographing system obtaining digital image data by means of Computed Radiography (hereinafter referred to as CR) , Magnetic Resonance Imaging (hereinafter referred to as MRI) or the like, and delivering the obtained digital image data to an image management apparatus and a display apparatus through a communication network (see, for example, Japanese Patent Application Publication (Unexamined) No. Tokukai 2000-139888).

Among all, the CR uses a phosphor plate composed of a photostimulable phosphor layer formed on a support member. In the CR, after a phosphor plate absorbs radiations transmitted through a patient, excitation light is irradiated to the phosphor plate to stimulate the radiation absorbed in the phosphor plate into light. Then, the stimulated light is photoelectrically converted. Thereby, an image signal is obtained.

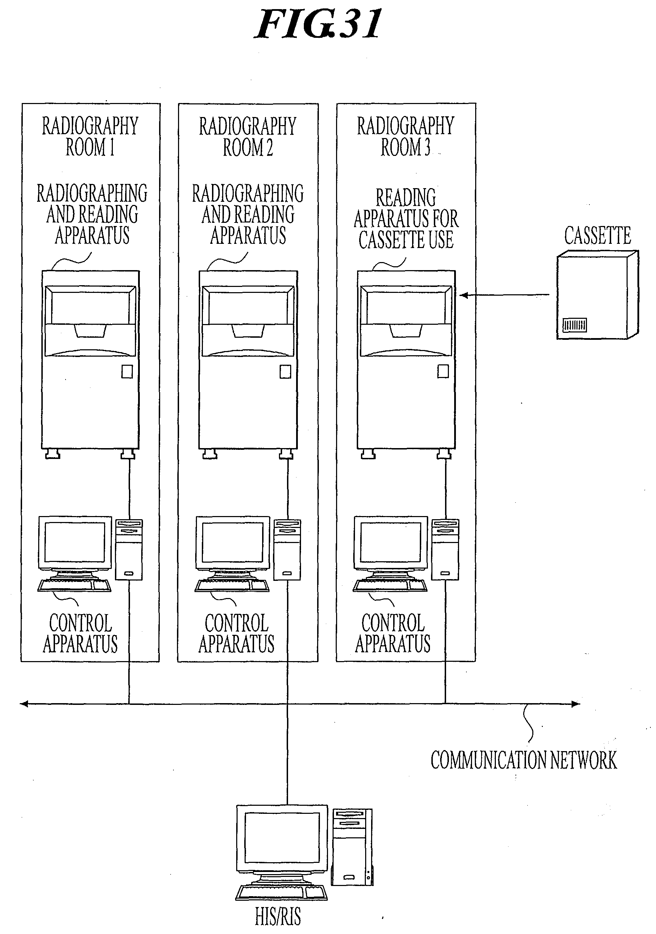

The medical image radiographing system will be described with reference to FIG. 31.

As shown in FIG. 31, in a conventional medical image radiographing system, generally a patient travels to a radiography room for performing radiography. In each radiography room, a radiographing-reading apparatus incorporating a phosphor plate therein for performing radiography and reading images, and a reading apparatus for cassette use for reading images out of a portable cassette containing a phosphor plate therein, and the like are fixedly installed.

Moreover, in each radiography room, a control apparatus is installed for controlling reading operation of images in the reading apparatus. These control apparatuses are connectable to a system for managing information in a hospital (hereinafter referred to as an HIS (Hospital Information System)), a system for managing the information in a department of radiology (hereinafter referred to as an RIS (Radiology Information System)), or the like through a communication network such as a local area network (LAN) or the like.

In the medical image radiographing system structured as described above, before radiography, according to a request from a doctor, the HIS or the RIS first issues radiographing order information including patient information such as a patient name, a sex, and the like of a patient to be radiographed; radiographing information such as a radiographic part, a radiographing method, and the like; examination information and the like. The issued radiographing order information is simultaneously delivered from the HIS or the RIS to all control apparatuses.

At the time of radiography, the radiographing order information is displayed on a monitor of the control apparatus, and an operator confirms the patient and the assigned radiographic part in the displayed radiographing order information to perform radiography. When radiography is performed with a radiographing-reading apparatus, the radiographed image is automatically read immediately after the radiography. On the other hand, when radiography is performed with a cassette, the operator inserts the cassette in the reading apparatus for cassette use, and has the reading apparatus read the radiographed image to obtain medical image data.

When a patient can travel to a radiography room, radiography is performed in the radiography room with the radiographing method described above. However, when it is difficult for a patient to travel to the radiography room, there is a case that radiography is performed at a patient's location such as the patient's bedside with a portable radiography apparatus (hereinafter referred to as a portable radiographing apparatus) and the cassette.

In a medical image radiographing system using the portable radiographing apparatus and the cassette, radiography performed by an operator is generally proceeded as follows:
(1) In the HIS, the RIS or the like, radiographing order information generated according to a request from a doctor is printed on an order form. Here, the radiographing order information includes the information related to a patient (hereinafter referred to as patient information) such as a name, a sex, and the like of a patient to be radiographed, and indicates a radiographing method and a patient to radiograph.
(2) The portable radiographing apparatus and the cassette are conveyed to the hospital room where the patient stays.
(3) After confirming the patient to be radiographed and a radiographic part based on the radiographing order information printed on the order form, the operator performs radiography with the portable radiographing apparatus and the cassette.
(4) The reading apparatus for cassette use reads the radiographed medical image out of the cassette.

However, in the radiographing method as described above, it is inconvenient that the operator has to bring the radiographing order information as the printed order form, i.e. a paper medium, to the patient's location. In particular, when a plurality of orders are generated to one patient, handling of the radiographing order information becomes so complicated that confusion of a combination between radiography to be performed and a cassette to be used for the radiography, could be caused. Here, the radiographing order information includes image process conditions for the radiographed medical image, and the medical image read out of a cassette after radiography is processed suitably for the radiographic part based on the radiographing order information. Therefore, when radiographing order information and a medical image are not properly related to each other, there is a possibility that a suitable image process is not performed and it causes decrease in diagnosis precision. Consequently, the handling of the radiographing order information requires accuracy and safety.

Moreover, in the conventional system structure, since identical radiographing order information is redundantly delivered from the HIS or the RIS to all the control apparatuses, there is a possibility that a plurality of operators confirm the identical radiographing order information on different control apparatuses, and then the plurality of operators simultaneously prepare the radiography to the same patient. In this case, the redundant preparation operations end up being a waste. Moreover, when radiography erroneously performed redundantly, the patient is needlessly exposed to radiation.

Moreover, the radiographing order information includes the patient information related to patient's privacy. In some cases, the radiographing order information includes important information such as a clinical diagnosis name or the like. Therefore, the leakage of the information to outside must be prevented. Therefore, in the medical image radiographing system, security should be ensured.

As a conventional art, a technique in which, when patient information is read, the identifier of a patient is read as well to specify whether or not it is the right patient, is proposed (see, for example, Japanese Patent Application Publication (Unexamined) No. Tokukai-hei 6-292657, pp. 2-4, FIG. 1).

However, in a case that the radiographing order information is displayed when a patient is specified by the identifier of the patient by using the conventional art technique for ensuring the security of the patient information included in the radiographing order information, the identifier has to be read at each radiography, and therefore radiography operation becomes complicated. Further, since only the radiographing order information of the patient whose identifier has been read is displayed, inconveniently, it is impossible to see the radiographing order information of a plurality of patients in a list.

### SUMMARY OF THE INVENTION

A first object of the present invention is to radiograph a medical image at a patient's location in a medical image radiographing system efficiently and accurately.

A second object of the present invention is to facilitate the handling of radiographing order information and prevent leakage of patient information to outside in a medical image radiographing system.

In accordance with a first aspect of the present invention, a medical image radiographing system comprises a radiographing apparatus for radiographing a medical image of a patient, an information management apparatus for managing radiographing order information, a control apparatus for obtaining the radiographing order information from the information management apparatus through a communication network, and a portable terminal connectable to the control apparatus, wherein the control apparatus comprises: a storage section for storing the radiographing order information obtained from the information management apparatus; and an assigning section for assigning an operator for performing radiography according to the radiographing order information stored, the control apparatus transmits the radiographing order information to the portable terminal along with operator identification information of the operator assigned, and the portable terminal comprises: a storage section for storing the radiographing order information and the operator identification information transmitted from the control apparatus; an authentication section for authenticating the operator on the basis of the operator identification information stored; and a display section for displaying the radiographing order information stored when the authentication section authenticates the operator.

In accordance with a second aspect of the present invention, a method for displaying radiographing order information in a medical image radiographing system comprising a radiographing apparatus for radiographing a medical image of a patient, an information management apparatus for managing radiographing order information, a control apparatus for obtaining the radiographing order information from the information management apparatus through a communication network, and a portable terminal connectable to the control apparatus, the method comprises: storing the radiographing order information obtained from the information management apparatus in a storage section of the control apparatus; assigning an operator for performing radiography of the radiographing order information stored; storing operator identification information of the operator assigned and the radiographing order information in a storage section of the portable terminal; authenticating the operator on the portable terminal on the basis of the operator identification information stored; and displaying the radiographing order information for which the operator is assigned on a display section of the portable terminal when the operator is authenticated.

According to the system of the first aspect or the method of the second aspect of the present invention, since the radiographing order information is displayed on the portable terminal, even when radiography is performed at a patient' s location such as a bedside of a patient or the like, an operator can easily confirm the patient to be radiographed and a radiographing condition with the portable terminal. Hence, medical image radiography at the patient's location can be efficiently performed. Moreover, since the operator is assigned and the radiographing order information is displayed when the portable terminal authenticates the operator, only the assigned operator can confirm the radiographing order information even when identical radiographing order information is delivered to a plurality of portable terminals. As a result, it is possible to prevent redundant radiography to the same patient. Consequently, it is possible to perform the medical image radiography at the patient's location accurately.

Preferably, in the system of the first aspect of the present invention, the portable terminal further comprises a warning section for warning that the operator is not assigned, when the authentication section does not authenticate the operator.

Preferably, the method of the second aspect of the present invention further comprises warning that the operator is not assigned when the operator is not authenticated.

According to the system or the method, since a warning is given when an operator is not authenticated, it is possible to draw operator's attention to assignation of the operator.

Preferably, the system of the first aspect further comprises a plurality of control apparatuses, wherein the portable terminal further comprises an input section for inputting radiography completion information for informing that the radiography according to the radiographing order information stored is completed, and transmits order identification information of the radiographing order information according to which the radiography has completed, to a control apparatus which has transmitted the radiographing order information to the portable terminal among the plurality of control apparatuses on the basis of the radiography completion information input, and each control apparatus transmits the order identification information of the radiographing order information transmitted from the portable terminal to the other control apparatuses, and when all the plurality of control apparatuses receive the order identification information of the radiographing order information from any one of the plurality of control apparatuses, all the plurality of control apparatuses delete the radiographing order information from the storage section.

Preferably, in the method of the second aspect, the medical image radiographing system further comprises a plurality of control apparatuses, the method further comprises: inputting radiography completion information at the portable terminal for informing that the radiography according to the radiographing order information is completed; transmitting order identification information of the radiographing order information from a control apparatus which has received the order identification information among the plurality of control apparatuses to the other control apparatuses among the plurality of control apparatuses based on the radiography completion information input, the radiographing order information according to which the radiography has completed; and deleting the radiographing order information according to which the radiography has completed from storage sections of the plurality of the control apparatuses when the plurality of control apparatuses receive the order identification information of the radiographing order information according to which the radiography has completed from any one of the plurality of control apparatuses.

According to the system or the method, since the control apparatus deletes the radiographing order information that another control apparatus regards as radiography-completed among radiographing order information stored therein, it is possible to prevent from delivering identical radiographing order information to a plurality of portable terminals, and thereby it is possible to improve process efficiency in the whole system as well as to reduce data amount stored in the control apparatus.

Preferably, in the above-mentioned system or in the above-mentioned method, the portable terminal is connectable to a specific control apparatus among the plurality of control apparatuses.

According to the system or the method, since the portable terminal is connected to the specific control apparatus, when system maintenance or a system change is performed, it is only necessary to do the maintenance on the specific control apparatus and the portable terminal connected to the control apparatus. Hence, it is possible to do the system change more flexibly and thereby it is easy to rebuild the system. Moreover, since the portable terminal is connected to the specific control apparatus, it is possible to stabilize the system.

Preferably, in the above-mentioned system or in the above-mentioned method, the portable terminal is connectable to any of the plurality of control apparatuses.

According to the system or the method, since the portable terminal is connectable to any one of the control apparatuses, any one of the control apparatus can transmit the radiographing order information to a desired portable terminal, and thereby user-friendliness of the system is improved.

In accordance with a third aspect of the present invention, a medical image radiographing system comprises: a control apparatus for storing radiographing order information including information related to a patient as downloadable, and capable of transmitting the radiographing order information to an external device in response to downloading instruction; a network connected to the control apparatus; a portable terminal for receiving the radiographing order information through the network and displaying the information related to the patient after authenticating an operator when providing the operator the radiographing order information; and a radiographing apparatus for performing radiography of the patient in accordance with instruction of the operator.

According to the system of the third aspect of the present invention, the portable terminal receives the radiographing order information from the information management apparatus through the network. Then after authenticating the operator, the portable terminal displays the information related to the patient included in the radiographing order information. As a result, the handling of the radiographing order information in the radiography operations becomes easy. Moreover, it is possible to prevent leakage of the information related to the patient. Consequently, it is possible to provide the medical image radiographing system having a high security function.

In accordance with a fourth aspect of the present invention, a portable terminal in a medical image radiographing system, comprises: a receiving section for receiving radiographing order information including information related to a patient; a display section for displaying the radiographing order information; an input section for inputting operator identification information of an operator; an identification information authentication section for authenticating the operator according to the operator identification information input at the input section; and a display control section for controlling display of the information related to the patient on the display section on the basis of a result of operator authentication by the identification information authentication section.

In accordance with a fifth aspect of the present invention, a display control method for displaying information related to a patient, comprises: receiving radiographing order information including the information related to a patient; displaying the radiographing order information on a display section, inputting operator identification information of an operator, authenticating the operator according to the operator identification information input, and controlling display of the information related to the patient on the display section on the basis of a result of the operator authentication.

In accordance with a sixth aspect of the present invention, a program makes a computer execute: receiving radiographing order information including information related to a patient; displaying the radiographing order information on a display section; receiving an input of operator identification information of an operator; authenticating the operator according to the operator identification information input; and controlling display of the information related to the patient on the display section on the basis of a result of the operator authentication.

Preferably, in the system of the third aspect of the present invention, the portable terminal comprises: a receiving section for receiving the radiographing order information including the information related to the patient, a radiographing order information storage section for storing the radiographing order information received by the receiving section, a display section for displaying the radiographing order information, an input section for inputting operator identification information of an operator, an identification information authentication section for authenticating the operator according to the operator identification information input at the input section, and a display control section for controlling display of the information related to the patient on the display section on the basis of a result. of operator authentication by the identification information authentication section.

According to the terminal of the fourth aspect, the method of the fifth aspect, the program of the sixth aspect, or the system of the present invention, since the portable terminal in the medical image radiographing system receives the radiographing order information and displays it, the confirmation of the radiographing order information and the handling thereof become easy. Thereby, labor of the operator on radiography operations can be reduced. Moreover, since the portable terminal controls display of the information related to the patient included in the radiographing order information on the basis of an authentication result of the input operator identification information, it is possible to prevent leakage of the information related to the patient to outside, and identity of the operator can be easily specified. Consequently, security in the medical image radiographing system can be improved.

Preferably, the terminal of the fourth aspect of the present invention further comprises a radiographing order information storage section for storing the radiographing order information received by the reception section, wherein the display control section controls display on the display section by downloading the information related to the patient from the radiographing order information storage section on the basis of the result of operator authentication by the identification information authentication section.

Preferably, the method of the fifth aspect of the present invention further comprises storing the radiographing order information received, wherein the controlling display of the information related to the patient includes controlling display on the display section by downloading the information related to the patient included in the radiographing order information stored on the basis of the result of the operator authentication.

Preferably, in the above-mentioned system, the display control section of the portable terminal controls display on the display section by downloading the information related to the patient from the radiographing order information storage section on the basis of the result of operator authentication by the identification information authentication section.

According to the terminal, the method or the system, the portable terminal stores the radiographing order information and, when controlling display thereon, the portable terminal downloads the stored radiographing order information to control the display thereof. Consequently, it is easy to download desired radiographing order information.

Preferably, in the terminal of the fourth aspect of the present invention, the display control section controls display on the display section by downloading the information related to the patient by way of a control apparatus from an information management apparatus managing the radiographing order information on the basis of the result of operator authentication by the identification information authentication section.

Preferably, in the method of the fifth aspect of the present invention, the controlling of the information related to the patient includes controlling display on the display section by downloading the information related to the patient by way of a control apparatus from an information management apparatus managing the radiographing order information on the basis of the result of the operator authentication.

Preferably, in the above-mentioned system, the display control section of the portable terminal controls display on the display section by downloading the information related to the patient by way of the control apparatus from the information management apparatus managing the radiographing order information on the basis of the result of operator authentication by the identification information authentication section.

According to the terminal, the method or the system, the portable terminal downloads the information related to the patient from the information management apparatus by way of the control apparatus on the basis of the authentication result of the operator identification information, and controls the display of the information related to the patient thereon. Consequently, not only in the portable terminal, by also in the information management apparatus, security can be ensured.

Preferably, in the terminal of the fourth aspect of the present invention, the identification information authentication section is set to operate in conjunction with a start-up of the portable terminal.

Preferably, in the method of the fifth aspect of the present invention, the authenticating the operator according to the identification information is done in conjunction with a start-up of the portable terminal.

Preferably, in the above-mentioned system, the identification information authentication section of the portable terminal is set to operate in conjunction with a start-up of the portable terminal.

According to the terminal, the method or the system, the operator identification information is authenticated in conjunction with the start-up of the portable terminal. Consequently, the security of the portable terminal handling the information related to the patient can be further improved.

In accordance with a seventh aspect of the present invention, a medical image radiographing system comprises: a control apparatus for managing radiographing order information and a medical image related to the radiographing order information; at least one portable terminal to which the control apparatus is capable of transmitting the radiographing order; an assigning section for assigning a portable terminal among the at least one portable terminal to which the control apparatus transmits the radiographing order information when the control apparatus transmits the radiographing order apparatus to the at least one portable terminal; and a communication control section for transmitting the radiographing order information to the portable terminal assigned.

Preferably, the system of the seventh aspect of the present invention further comprises a storage section for storing an address allocated for each of the at least one portable terminal; wherein the assigning section assigns the portable terminal to which the control apparatus transmits the radiographing order information to with the address; and the communication control section controls communication of the system with the address.

More preferably, in the above-mentioned system, the control apparatus comprises the assigning section.

Preferably, the system of the seventh aspect of the present invention further comprises a plurality of control apparatuses, wherein the plurality of control apparatuses are connected to each other through a network.

More preferably, in the above-mentioned system, the at least one portable terminal is capable of communicating with the plurality of control apparatuses through the network.

More preferably, in the above-mentioned system, the at least one portable terminal is capable of communicating with a predetermined control apparatus among the plurality of control apparatuses.

In accordance with an eighth aspect of the present invention, a medical image radiographing system comprises: a radiographing order generating apparatus for generating radiographing order information; a control apparatus for obtaining the radiographing order information and relatedly managing the radiographing order information and at least one of information related to the radiographing order information and a medical image; at least one portable terminal to which the control apparatus is able to transmit the radiographing order; an assigning section for assigning a portable terminal to which the control apparatus transmits the radiographing order information among the at least one portable terminal when the control apparatus transmits the radiographing order information to the at least one portable terminal; and a communication control section for transmitting the radiographing order information to the portable terminal assigned.

Preferably, the system of the eighth aspect of the present invention further comprises a storage section for storing an address allocated for each of the at least one portable terminal, wherein the assigning section assigns the portable terminal to which the control apparatus transmits the radiographing order information with the address, and the communication control section controls communication of the system with the address.

More preferably, in the above-mentioned system, the control apparatus comprises the assigning section.

Preferably, the system of the eighth aspect of the present invention further comprises a plurality of control apparatuses, wherein the plurality of control apparatuses are connected to each other through a network.

More preferably, in the above-mentioned system, the at least one portable terminal is capable of communicating with the plurality of control apparatuses through the network.

More preferably, in the above-mentioned system, the at least one portable terminal is capable of communicating with a predetermined control apparatus among the plurality of control apparatuses.

In accordance with a ninth aspect of the present invention, a medical image radiographing system comprises: at least one control apparatus for managing radiographing order information and a medical image related to the radiographing order information; at least one portable terminal comprising at least one of a receiving section for receiving the radiographing order information from the at least one control apparatus, and a transmitting section for transmitting a radiography result corresponding to the radiographing order information; an assigning section for assigning at least one of a portable terminal among the at least one portable terminal to which the at least one control apparatus transmits the radiographing order information when the at least one control apparatus transmits the radiographing order information to the at least one portable terminal, and a control apparatus among the at least one control apparatus to transmit the radiography result to when the at least one portable terminal transmits the radiography result to the at least one control apparatus; a communication control section, when the assigning section assigns a portable terminal among the at least one portable terminal to transmit the radiographing order to, for transmitting the radiographing order information to the portable terminal, and, when the assigning section assigns a control apparatus among the at least one control apparatuses to transmit the radiography result to, for transmitting the radiography result to the control apparatus.

Preferably, the system of the ninth aspect of the present invention further comprises a storage section for storing an address allocated for each of the at least one portable terminal, wherein the assigning section assigns a portable terminal among the at least one portable terminal to transmit the radiographing order information to with the address, and the communication control section controls communication of the medical image radiographing system with the address.

Preferably, the system of the ninth aspect of the present invention further comprises a storage section for storing an address allocated for each of the at least one control apparatus, wherein the assigning section assigns a control apparatus among the at least one control apparatus to transmit the radiographing order information according to which radiography has been completed to with the address, and the communication control section controls communication of the medical image radiographing system with the address.

More Preferably, in the above-mentioned system, the storage section stores an address allocated for each of the at least one control apparatus, and the assigning section assigns a control apparatus among the at least one control apparatus to transmit the radiographing order information according to which radiography has been completed to with the address.

More preferably, in the above-mentioned system, the at least one control apparatus comprises the assigning section.

Preferably, in the system of the ninth aspect of the present invention, each of the at least one control apparatus and the at least one portable terminal comprises the assigning section and the communication control section.

Preferably, the system of the ninth aspect of the present invention further comprises a plurality of control apparatuses, wherein the plurality of control apparatuses are connected to each other through a network.

In accordance with a tenth aspect of the present invention, a medical image radiographing system comprises: a radiographing apparatus for radiographing a medical image of a patient; an information management apparatus for managing radiographing order information and transmitting the radiographing order information to outside according to instruction through a communication network; a plurality of control apparatuses for generating a radiographing order list by receiving the radiographing order information from the information management apparatus through the communication network, transmitting the radiographing order information assigned in the radiographing order list to a portable terminal and relating the radiographing order information with radiography result related information transmitted from the portable terminal; and at least one portable terminal connectable to at least one of the plurality of control apparatuses for transmitting the radiography result related information corresponding to the radiographing order information received from the at least one of the plurality of control apparatuses to all the plurality of control apparatuses, wherein when one of the plurality of control apparatuses relates the radiographing order information with the radiography result related information, the radiographing order information is deleted from the radiographing order list of the other control apparatuses.

Preferably, in the system of the tenth aspect of the present invention, the radiographing apparatus radiographs a radiation image by using a cassette, and the radiography result related information is identification information of the cassette used for radiographing the radiation image.

In accordance with an eleventh aspect of the present invention, a medical image radiographing system comprises: a radiographing apparatus for radiographing a medical image of a patient, an information management apparatus for managing radiographing order information and transmitting the radiographing order information to outside according to instruction through a communication network, a plurality of control apparatuses for generating a radiographing order list by receiving the radiographing order information from the information management apparatus through the communication network, transmitting the radiographing order information assigned in the radiographing order list to a portable terminal and relating the radiographing order information with radiography result related information transmitted from the portable terminal; and at least one portable terminal connectable to at least one of the plurality of control apparatuses for displaying the radiographing order information received from the at least one of the plurality of control apparatuses; wherein when any one of the plurality of control apparatuses transmits the radiographing order information to any one of the at least one portable terminal, the radiographing order information is deleted from the radiographing order list of the other control apparatuses.

### BRIEF DESCRIPTION OF THE DRAWINGS

The present invention will become more fully understood from the detailed description given hereinafter and the accompanying drawing given by way of illustration only, and thus are not intended as a definition of the limits of the present invention, and wherein:
FIG. 1 is a view showing a system structure of a medical image radiographing system 100 of a first embodiment of the present invention,
FIG. 2 is a view showing a functional structure of a control apparatus 140,
FIG. 3 is a view showing a data structure of a radiographing order information file 1461,
FIG. 4 is a view showing a data structure of a registered information file 1462,
FIG. 5 is a view showing a functional structure of a portable terminal 160,
FIG. 6 is a flowchart illustrating a radiography preparation process executed by the control apparatus 140,
FIG. 7A is a view showing a menu screen 1431a when normal radiography is selected, and FIG. 7B is a view showing a menu screen 1431b when portable radiography is selected,
FIG. 8 is a view showing a patient list screen 1432,
FIG. 9 is a flowchart illustrating a new registration process executed by the control apparatus 140,
FIG. 10 is a view showing an input screen 1433 for inputting patient information,
FIG. 11 is a view showing a selection screen 1434 for selecting a radiographing condition,
FIG. 12 is a flowchart illustrating an order transmission process executed by the control apparatus 140,

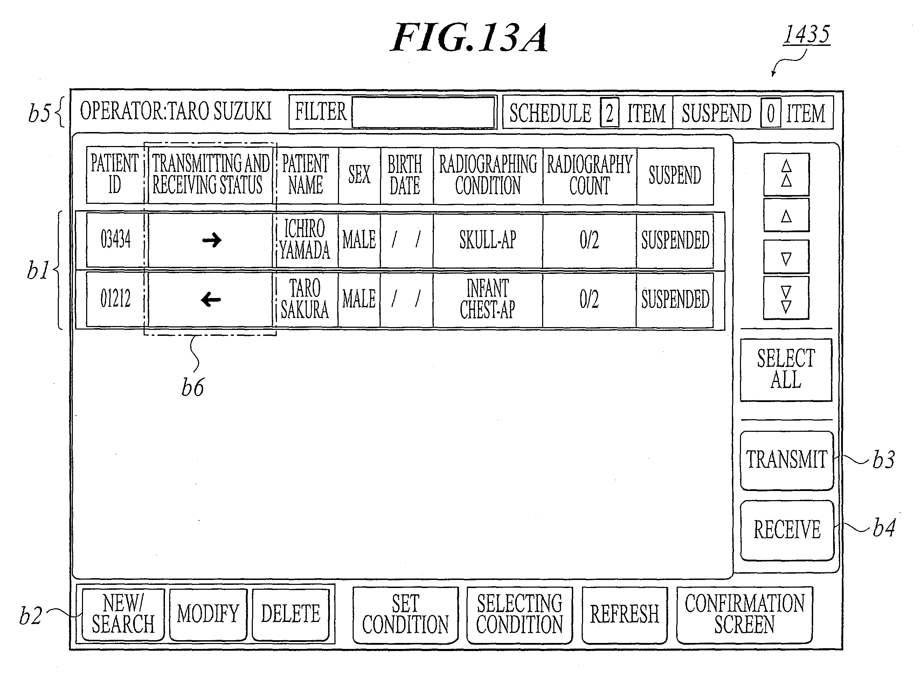

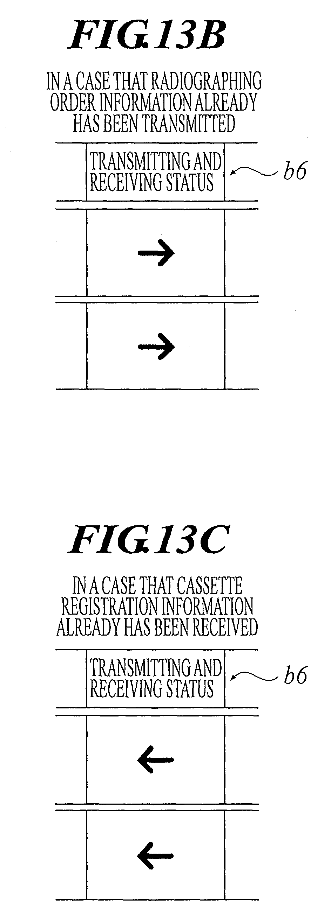

FIG. 13A is a view showing a confirmation screen 1435 for confirming the transmitting and receiving status of radiographing order information, FIG. 13B is a view showing marks displayed in a transmitting and receiving status b6 in a case that the control apparatus 140 already has transmitted the radiographing order information a portable terminal 160, and FIG. 13C is a view showing marks displayed in the transmitting and receiving status b6 in a case that the control apparatus 140 already has received cassette registration information corresponding to the transmitted radiographing order information from the portable terminal 160,
FIG. 14A and 14B are flowcharts illustrating a cassette registration process executed by the portable terminal 160,
FIG. 15 is a view showing an authentication screen 1631 for authenticating an operator,
FIG. 16A is a view showing a patient list screen 1632, and FIG. 16B is a view showing an order list screen 1633,
FIG. 17 is a flowchart illustrating an image registration process executed by the control apparatus 140,
FIG. 18 is a flowchart illustrating a radiographed order process executed by the control apparatus 140,
FIG. 19 is a view showing an image confirmation screen 1437,
FIG. 20 is a view showing a system structure of a medical image radiographing system 100' of another embodiment of the present invention,
FIG. 21 is a conceptual diagram showing a whole structure of a medical image radiographing system 200 including a PDA 210 of a second embodiment of the present invention,
FIG. 22 is a block diagram showing a functional structure of the PDA 210 in the medical image radiographing system 200,
FIG. 23 is a view showing an example of storing data in an operator ID table 2161 of a storage 216 in FIG. 22,
FIG. 24 is a view showing an example of storing data in an order registration file 2162 of the storage 216 in FIG. 22,
FIG. 25 is a view illustrating an overall flow of radiography performed with the PDA 210,
FIG. 26 is a flowchart illustrating an order authentication process executed by a CPU 211 in FIG. 22,
FIG. 27 is a view showing an example of a list screen 2131 of the radiographing order information displayed when an operator is authenticated in the order authentication process,
FIG. 28 is a view showing an example of detail screen 2132 for displaying detailed patient information,
FIG. 29 is a view showing a system structure of a medical image radiographing system 300 as one of modified examples of the medical image radiographing system 200,
FIG. 30A and 30B are flowcharts illustrating radiography processes appropriate to both the connection types between a portable terminal and a control apparatus in a medical image radiographing system capable thereof, and
FIG. 31 is a view showing a system structure of a medical image radiographing system in a conventional art.

### EMBODIMENTS OF THE INVENTION

### [First Embodiment]

Hereinafter, with reference to figures, a first embodiment of the present invention will be explained in detail.

In the first embodiment, an example of a structure where a medical image radiographing system comprises a portable terminal for displaying radiographing order information at a patient's location, an operator performing radiography is assigned in a control apparatus, identification information of the assigned operator is related with the radiographing order information and transmitted from the control apparatus to the portable terminal, and in the portable terminal, the radiographing order information is displayed only when the operator is authenticated by performing the authentication, will be explained.

First, a structure of the first embodiment will be described.

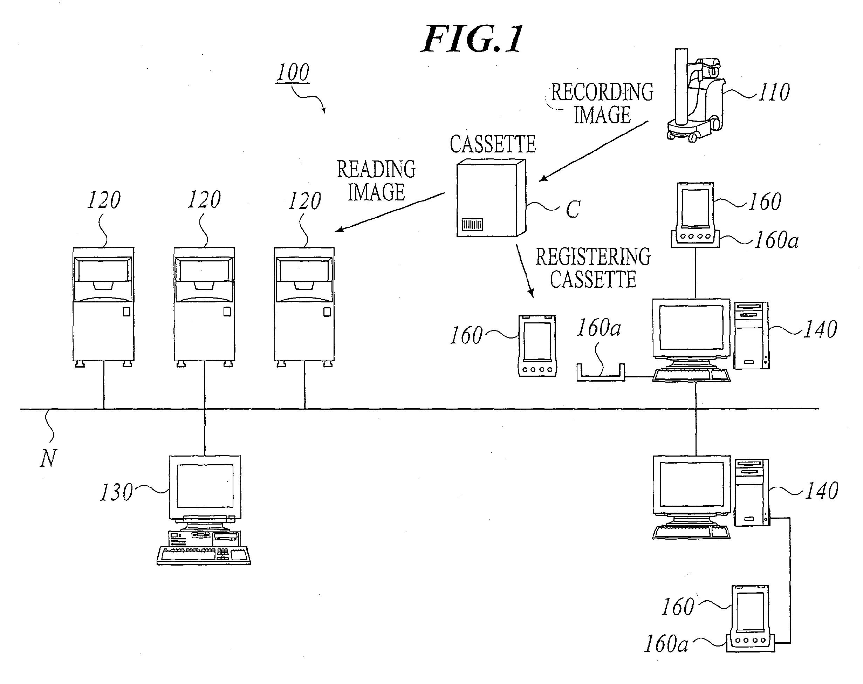

FIG. 1 shows a system structure of the medical image radiographing system 100 in the first embodiment.

As shown in FIG. 1, the medical image radiographing system 100 comprises a portable radiographing apparatus 110, a reading apparatus 120 for cassette use, an information management apparatus 130, a control apparatus 140, and a portable terminal 160. The reading apparatus 120, the information management apparatus 130 and the control apparatus 140 are connected to each other through a communication network N. Moreover, the portable terminal 160 is connected to a specific control apparatus 140 via a communication terminal 160a in a state capable of transmitting and receiving information. Here, the numbers of the installed control apparatus 140 and the portable terminals 160 may be any, and are not specifically limited.

The portable radiographing apparatus 110, which is a movable radiographing apparatus equipped with a radiation source, radiographs a patient at a patient's location (hereinafter referred to as portable radiography). The portable radiographing apparatus 110 records the radiographed medical image in a cassette c. The cassette c incorporates a phosphor plate therein, and records the medical image radiographed by the portable radiographing apparatus 110. Cassette identification information (hereinafter referred to as cassette ID) is provided as a barcode on the surface of the cassette c for distinguishing itself from the other cassettes c.

The reading apparatus 120 reads the medical images recorded in the cassettes c in accordance with instruction from the control apparatus 140. The reading apparatus 120 transmits the medical images read out of the cassette c and the cassette ID of the cassette c to the control apparatus 140.

The information management apparatus 130 accepts a request from a doctor, and issues radiographing order information based on the request. The information management apparatus 130 manages the issued radiographing order information with order identification information (hereinafter referred to as order ID) added for distinguishing the radiographing order information from the other radiographing order information. The information management apparatus 130 may be an entry terminal for accepting radiographing order to issue radiographing order information or an information management system such as an HIS, an RIS or the like.

The information management apparatus 130 simultaneously delivers the issued radiographing order information to each control apparatus 140, and receives transmit information of certain radiographing order information and, in case, radiographing completion information related thereto from each control apparatus 140, for managing the radiographing order information and obtained medical images. Here, the transmit information is information indicating that radiographing order information related thereto has already transmitted to any one of the portable terminals 160.

The control apparatus 140 controls the reading operation of the medical images in the reading apparatus 120, and receives the read medical image from the reading apparatus 120 to reconfirm and relate the radiographing order information with the medical images (hereinafter referred to as image registration).

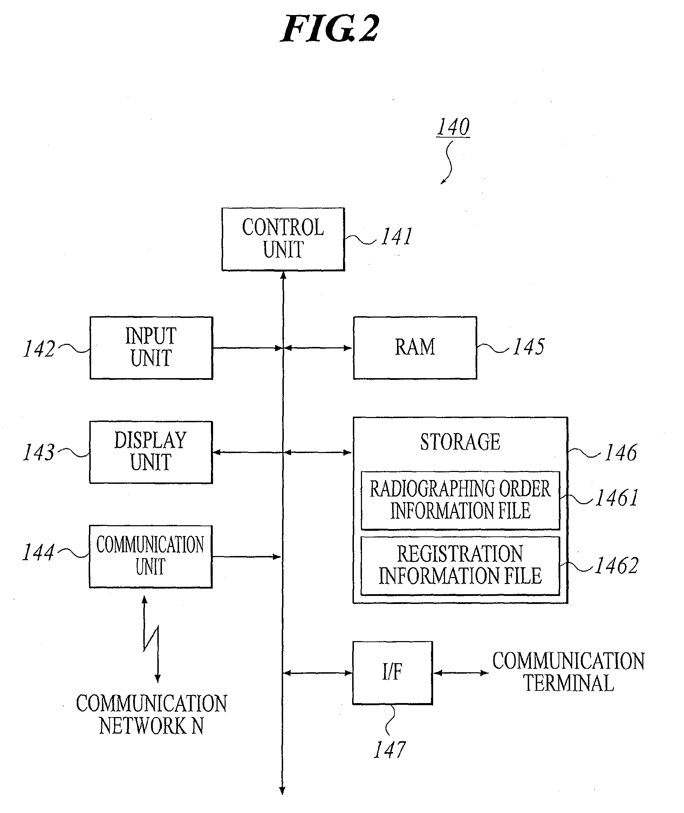

FIG. 2 shows a functional structure of a control apparatus 140.

As shown in FIG. 2, the control apparatus 140 comprises a control unit 141, an input unit 142, a display unit 143, a communication unit 144, a RAM (random access memory) 145, a storage 146 and an interface (I/F) 147.

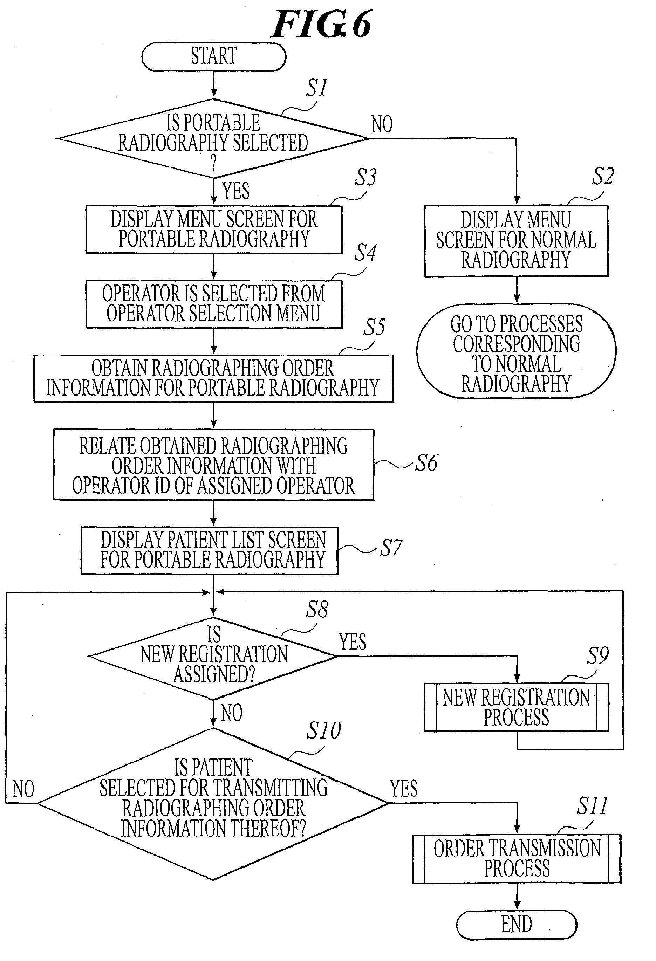

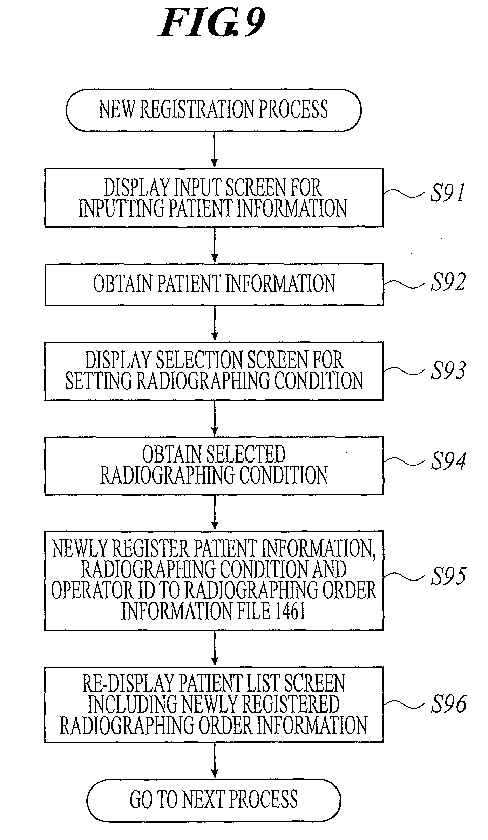

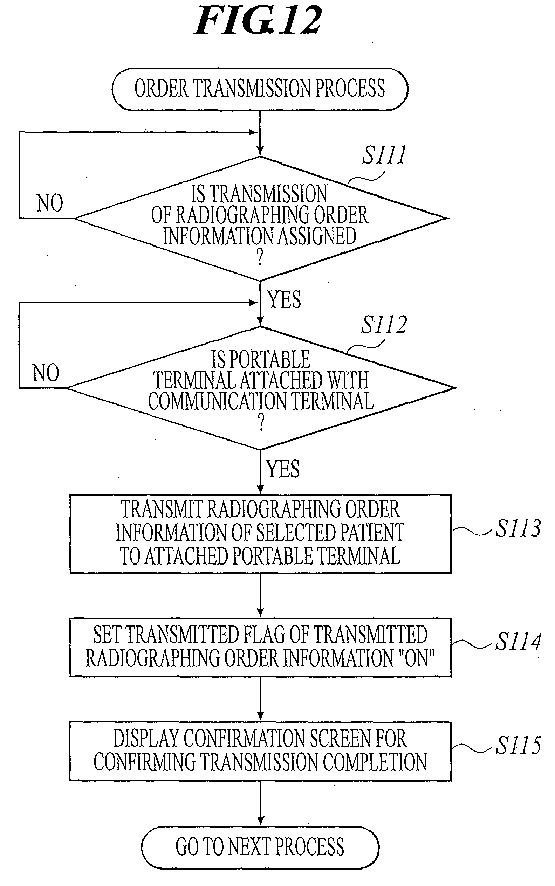

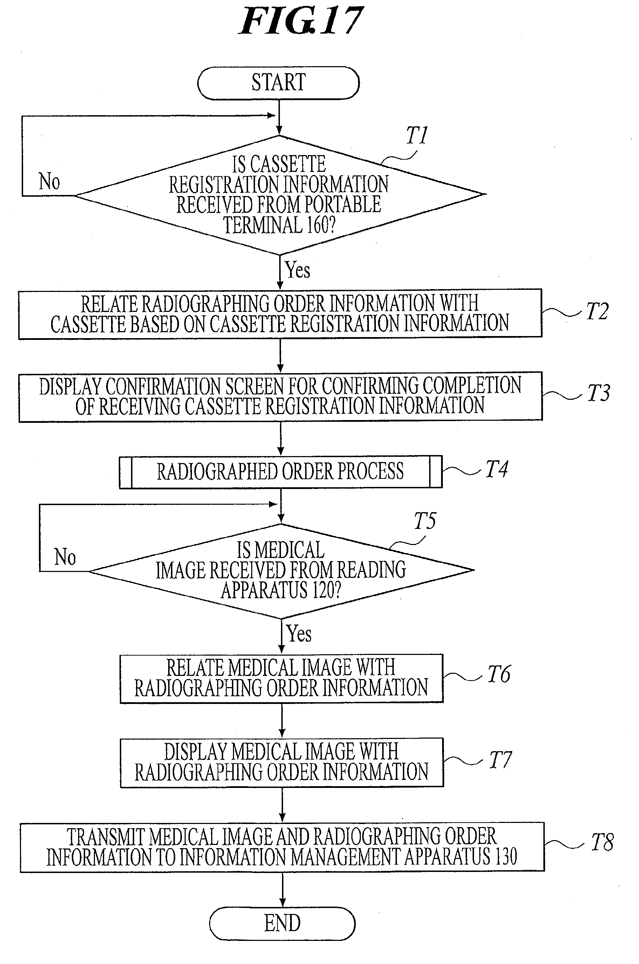

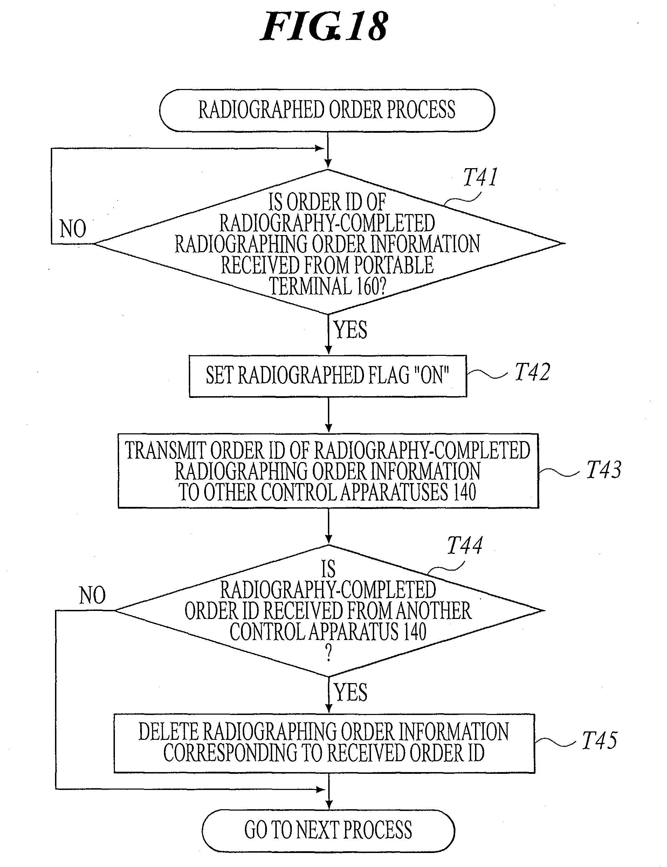

The control unit 141 is composed of a central processing unit (CPU) and the like. The control unit 141 develops programs such as a system program stored in the storage 146, a radiography preparation process program (see FIG. 6), a new registration process program (see FIG. 9), an order transmission process program (see FIG. 12), an image registration process program (see FIG. 17), a radiographed order process program (see FIG. 18) and the like, into the RAM 145, and integrally controls the process operation in cooperation with the programs.

The input unit 142 is composed of a keyboard including numeric keys, letter keys, various function keys and the like, or a touch panel integrated with the display unit 143, for inputting patient information and radiographing information, or for assigning an operator. The input unit 142 outputs an operation signal corresponding to an operated key to the control unit 141.

The display unit 143, composed of a liquid crystal display (LCD) and the like, displays various operation screens and various display information such as process results of the control unit 141 and the like.

The communication unit 144 is composed of a network interface, a modem and the like. The communication unit 144 transmits and receives information with an external device connected to the communication network N. The communication unit 144 receives radiographing order information from the information management apparatus 130.

The RAM 145 forms a work area for temporarily storing various programs to be executed by the control unit 141 and data pertaining to the programs.

The storage 146 is composed of a magnetic storage medium, an optical storage medium, or a semiconductor memory. The storage 146 stores the system program, the radiography preparation process program, the new registration process program, the order transmission process program, the image registration process program, the radiographed order process program, the data processed by each program and the like.

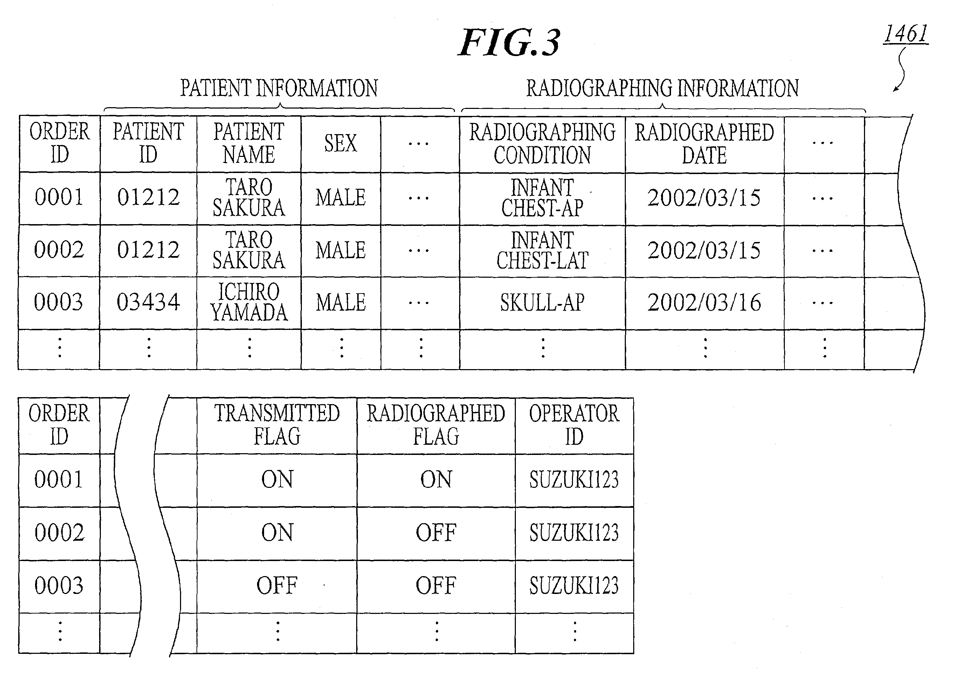

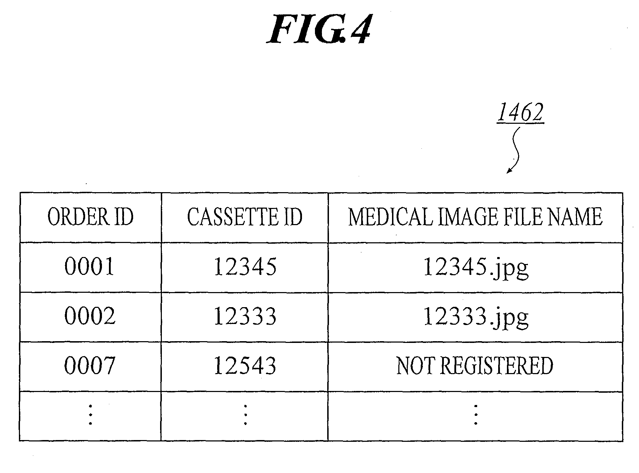

The storage 146 comprises an image database (not shown) , and stores data files of medical images received from the reading apparatus 120. Moreover, the storage 146 comprises a radiographing order information file 1461 (see FIG. 3) for storing the radiographing order information received from the information management apparatus 130 as updateable, a registered information file 1462 (see FIG. 4) for storing correspondence relation between the radiographing order information and radiographed medical images, and an operator list (not shown) in which operator names and operator identification information (hereinafter referred to as operator ID) for distinguishing the operator from the other operators are related to each other.

In the radiographing order information file 1461, as shown in FIG. 3, the radiographing order information is stored in the turn of its order ID, which are able to be stored. The radiographing order information includes the information related to a patient to be radiographed such as a patient ID, a patient name, and the like (hereinafter referred to as patient information), the information related to radiography such as a radiographing condition, a radiographed date and the like (hereinafter referred to as radiographing information), and the like. Moreover, in each radiographing order information, there are a transmitted flag indicating whether the radiographing order information has been transmitted to the portable terminal 160 or not, and a radiographed flag indicating whether the radiography corresponding to the radiographing order information has been performed or not. The transmitted flag in the radiographing order information which has been transmitted to the portable terminal 160 is set "ON", and the transmitted flag in the radiographing order information which has not been transmitted yet is set "OFF". Similarly, the radiographed flag in the radiographing order information of the radiography which has been performed is set "ON", and the radiographed flag in radiographing order information of the radiography which has not been performed is set "OFF". Moreover, the operator ID of the operator who performs radiography is related to the radiographing order information.

As shown in FIG. 4, the registered information file 1462 relatedly stores the order ID of the radiographing order information, the cassette ID of the cassette c used for the radiography based on the radiographing order information, and the file name of the medical image read out of the cassette c.

The I/F 147 is an interface for connecting between the control apparatus 140 and the communication terminal 160a. When the portable terminal 160 is attached to the communication terminal 160a, the I/F 147 outputs a detection signal to the control unit 141. The I/F 147 transmits radiographing order information to the portable terminal 160 via the communication terminal 160a, and receives the cassette registration information according to the transmitted radiographing order information from the portable terminal 160.

In case that several pairs of control apparatuses 140 and portable terminals 160 are provided in the system, when one control apparatus 140 transmits radiographing order information to one portable terminal 160, the other control apparatuses 140 may be managed not to transmit the radiographing order information.

The other control apparatuses 140 receive transmit information of certain radiographing order information from one control apparatus 140 or the information management apparatus 130, the radiographing order information is deleted on each display of the other control apparatuses 140.

Next, the portable terminals 160 will be described.

A portable terminal 160 is a portable information processing device connectable to a specific control apparatus 140 via a communication terminal 160a. The portable terminal 160 receives radiographing order information for portable radiography from the control apparatus 140, and displays the received radiographing order information. Moreover, the portable terminal 160 relates the radiographing order information with the cassette c to be used for radiography (hereinafter referred to as cassette registration), and transmits the cassette registration information indicating the correspondence relation to the control apparatus 140.

The communication terminal 160a is connected to a specific control apparatus 140 through a cable or the like, and controls the transmission and the reception of information between the portable terminal 160 attached thereto and the specific control apparatus 140. Moreover, when the portable terminal 160 is attached thereto, the communication terminal 160a transmits a detection signal to the control apparatus 140, and starts charging the attached portable terminal 160.

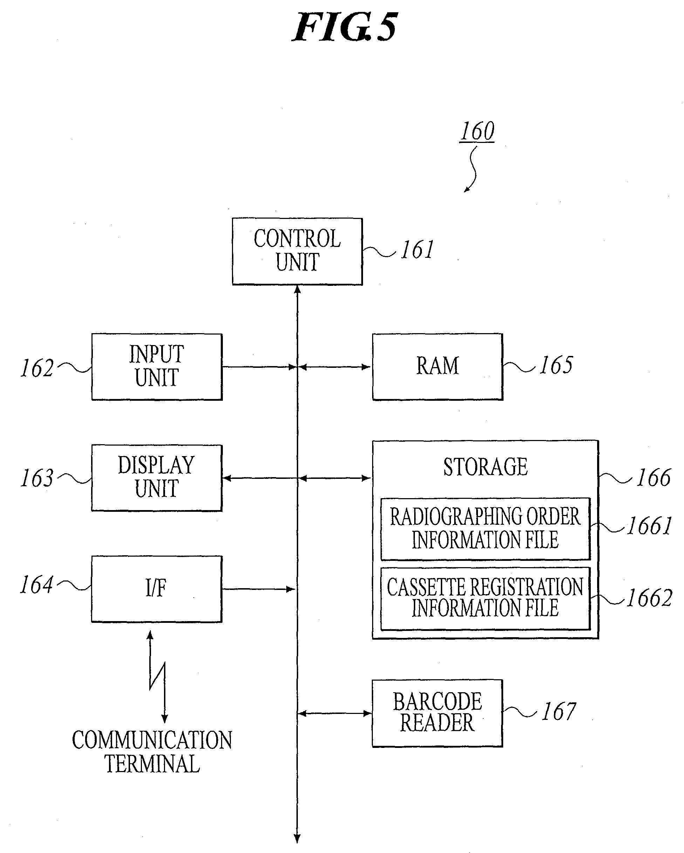

FIG. 5 shows a functional structure of the portable terminal 160.

As shown in FIG. 5, the portable terminal 160 comprises a control unit 161, an input unit 162, a display unit 163, an I/F 164, a RAM 165, a storage 166 and a barcode reader 167.

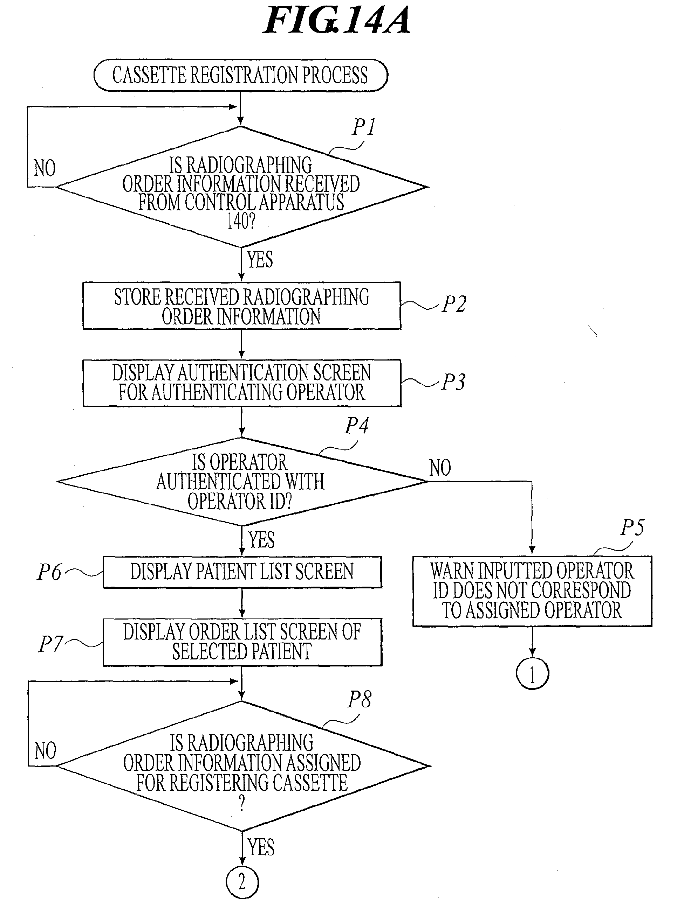

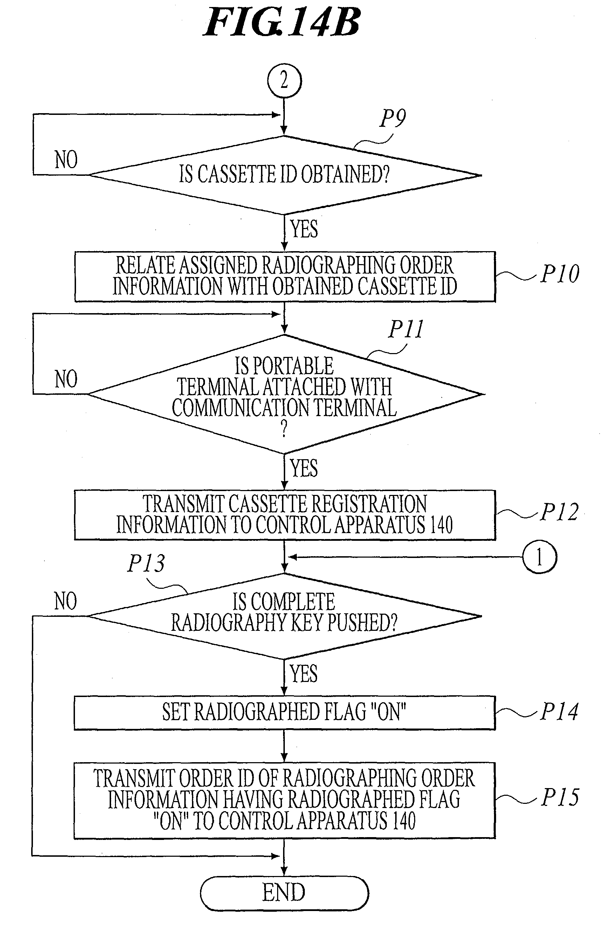

The control unit 161 is composed of a CPU and the like. The control unit 161 develops a system program stored in the storage 166, a cassette registration process program (see FIG. 14) and the like according to the present invention into the RAM 165, and integrally controls process operations of the portable terminal 160 in cooperation with the programs.

In a cassette registration process, the control unit 161 collates the operator ID of the operator assigned in radiographing order information with the operator ID input by an operator at the input unit 162 for authenticating the operator.

The input unit 162 is composed of a keyboard including numeric keys, letter keys, various function keys and the like, or a touch panel, a jog dial and the like, which are integrated with the display unit 163. In other words, with these keyboards, the touch panel and the jog dial, the operator can input the selection of the radiographing order information, the operator ID, and the radiography completion information for informing the radiography completion.

The display unit 163 is composed of an LCD and the like. The display unit 163 displays various operation screens and various display information such as the input information at the input unit 162, process results of the control unit 161 and the like. Moreover, in the cassette registration process, when an operator is not authenticated, the display unit 163 displays a warning message for warning that the input operation ID does not correspond to the assigned operator.

The I/F 164 is an interface for connecting the portable terminal 160 with the communication terminal 160a. When the portable terminal 160 is attached to the communication terminal 160a, the I/F 164 outputs a detection signal to the control unit 161. Moreover, when the I/F 164 receives radiographing order information from the control apparatus 140 via the communication terminal 160a, the I/F 164 transmits the cassette registration information corresponding to the received radiographing order information to the control apparatus 140. Here, the I/F 164 may be connected to a portable telephone terminal such as a PHS or the like to establish radio communication for transmitting and receiving data as the need arises.

The RAM 165 forms a work area for temporarily storing various programs to be executed by the control unit 161 and data pertaining to the programs.

The storage 166 is composed of a magnetic storage medium, an optical storage medium, or a semiconductor memory. The storage 166 stores the system program, the cassette registration process program, the data processed by each program, and the like.

Moreover, the storage 166 comprises an radiographing order information file 1661 storing the radiographing order information received from the control apparatus 140 as updateable, and a cassette registration information file 1662 for registering the correspondence relation between the radiographing order information and the cassette c used for radiography corresponding thereto.

Since the data structure of the radiographing order information file 1661 is approximately the same as that of the radiographing order information file 1461 in the control apparatus 140 as described above, only except that the radiographing order information file 1461 is lack of the transmitted flag, the illustration and the description of the radiographing order information file 1661 is hereupon omitted. In other words, in the radiographing order information file 1661, a plurality of radiographing order information is stored in the turn of its order ID and the radiographed flag and the operator ID of the assigned operator are related with each radiographing order information.

Since the data structure of the cassette registration information file 1662 is approximately the same as that of the registered information file 1462 the control apparatus 140 comprises as described above, only except that the registered information file 1462 is lack of the medical image file name, the illustration and the description of the cassette registration information file 1662 is hereupon omitted. In other words, in the cassette registration information file 1662, the order ID of the radiographing order information and the cassette ID of the cassette c used for radiography corresponding thereto are relatedly stored.

The barcode reader 167, including a scanner having an optical read mechanism, reads the barcode of the cassette ID on the surface of the cassette c, and obtains the cassette ID indicated by the barcode by decoding the read barcode in accordance with a predetermined standard. Here, it is also possible to attach the barcode of the patient ID with the bedside of the patient or a part of the body of the patient, and to read the accompanied barcode with the barcode reader 167 for obtaining the patient ID.

Next, operations of the first embodiment will be described.

First, with reference to FIG. 6, the radiography preparation process executed by the control apparatus 140 will be described. In the radiography preparation process, when portable radiography is performed at a patient's location, an operator is assigned as a person who performs the radiography, and the radiographing order information of the portable radiography is related to the operator ID of the assigned operator to be transmitted to the portable terminal 160.

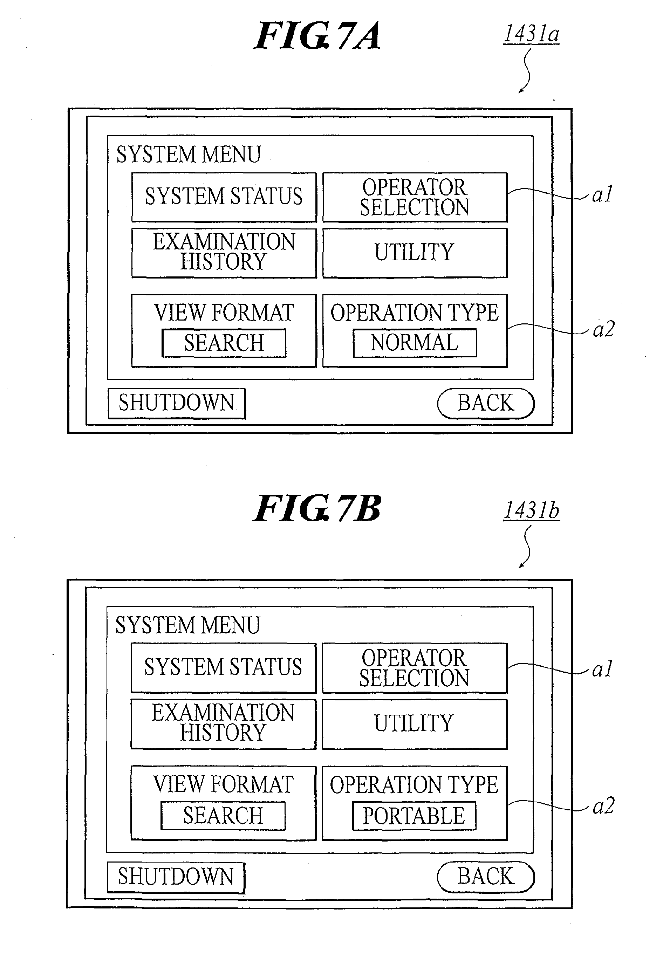

In the radiography preparation process shown in FIG. 6, at first in Step S1, guidance to select an operation type between normal radiography with the radiographing-reading apparatus fixedly installed in a radiography room, or portable radiography with the portable radiographing apparatus 110 and the cassette c at the patient's location for performing radiography, is given. Then, which operation type has been selected and input at the input unit 142 is judged. When the normal radiography is selected (Step S1; NO), a menu screen 1431a (see FIG. 7A) for the normal radiography is displayed on the display unit 143 (Step S2), and the operation of the control apparatus 140 proceeds to the processes corresponding to the normal radiography. As shown in FIG. 7A, on the menu screen 1431a, menu keys for selecting various menus such as an "OPERATOR SELECTION" menu key a1 for selecting an operator, an "OPERATION TYPE" menu key a2 for proceeding to the radiography preparation process and the like are displayed, and the "OPERATION TYPE" menu key a2 shows that the radiography preparation process is to be performed in a normal radiography mode.

On the other hand, when the portable radiography is selected (Step S1; YES), a menu screen 1431b (see FIG. 7B) for the portable radiography is displayed (Step S3) . As shown in FIG. 7B, on the menu screen 1431b, various menu keys are displayed similarly to the menu screen 1431a. The "OPERATION TYPE" menu key a2 shows that the radiography preparation process is to be performed in a portable radiography mode. When an operator who performs the portable radiography needs to be assigned, the "OPERATOR SELECTION" menu key a1 is pushed down. When the radiography preparation is performed after the operator is assigned, the "OPERATION TYPE" menu key a2 is pushed down.

When the "OPERATOR SELECTION" menu key a1 is pushed down on the menu screen 1431b, a selection screen (not shown) on which operators are shown as a list on the basis of the operator list stored in the storage 146 is displayed where one can be selected among the displayed operators on the display unit 143 in Step S4, and guidance to select an operator to be assigned is given. The operator selects for assigning an operator who performs the radiography among the operators displayed in the list on the selection screen.

After the operator is selected and assigned at the input unit 142, the menu screen 1431a is re-displayed on the display unit 143. Then, when the "OPERATION TYPE" menu key a2 is pushed down on the re-displayed menu screen 1431a, request information for requesting radiographing order information for portable radiography to the information management apparatus 130 is generated in Step S5, and the request information is transmitted to the information management apparatus 130 by the communication unit 144. Then, when there is the radiographing order information for portable radiography of which radiography has not yet been performed left in the information management apparatus 130, the control apparatus 140 obtains the radiographing order information from the information management apparatus 130.

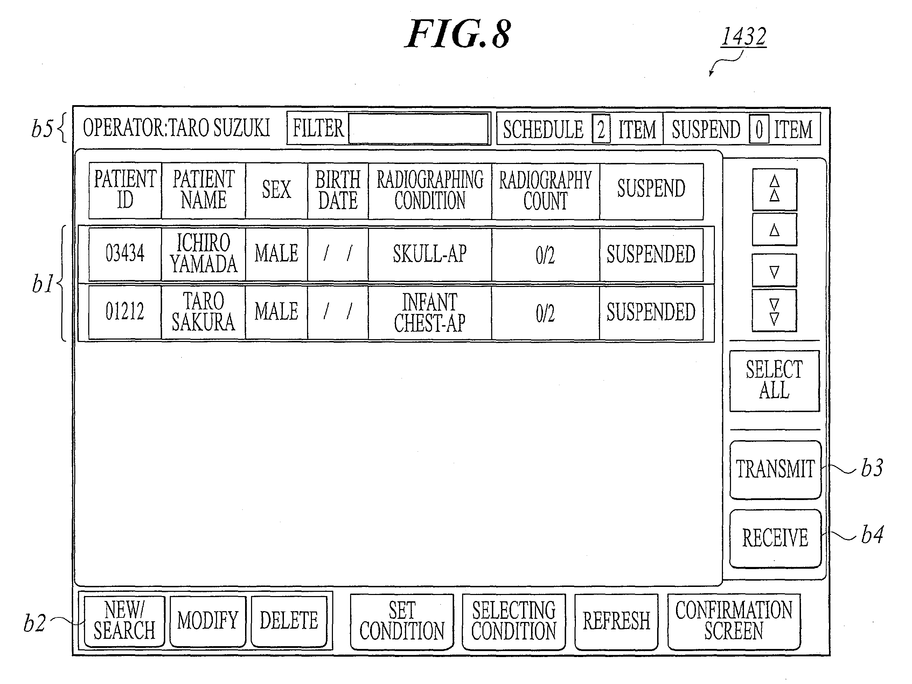

Next, in Step S6, the obtained radiographing order information is related to the operator ID of the assigned operator to be stored in the radiographing order information file 1461. Then, in Step S7, a patient list screen on which the patients to be radiographed are displayed as a list is displayed on the basis of the obtained radiographing order information. In this case, when the radiographing order information is obtained from the information management apparatus 130, a patient list screen 1432 in which the patient names of the patients to be radiographed, the patient IDs and the like are shown as a list in display area b1 is displayed as shown in FIG. 8. When there is no radiographing order information obtained from the information management apparatus 130 left, the screen display of the patient list screen 1432 is displayed with the display area b1 displaying no patient list therein.

As shown in FIG. 8, in the patient list screen 1432, a patient ID, a patient name, a sex, a radiographic part, radiography count and the like of the patients to be radiographed are displayed in the display area b1 at each of the patients. When an operator is assigned, the name of the assigned operator is displayed in a display area b5. Moreover, in the lower part of the screen, a new/search key b2 for newly registering radiographing order information and for searching the radiographing order information with a patient name among the displayed patient names as a search key, is displayed. On the right side of the screen, a transmission key b3 for instructing to transmit radiographing order information and a reception key b4 for instructing to receive the cassette registration information corresponding to the radiographing order information from the portable terminal 160 are displayed. When an operator wants to register radiographing order information newly, the operator pushes down the new/search key b2. When the operator wants to transmit radiographing order information to the portable terminal 160, the operator selects a patient of the corresponding radiographing order information among the patients displayed in the list. Then, after the operator attached the portable terminal 160 to the communication terminal 160a, the operator pushes the transmission key b3 in the patient list screen 1432.

Next, in Step S8, whether the new registration of the radiographing order information has been assigned by means of the new/search key b2 or not is judged. In a case that the new registration of the radiographing order information has been assigned (Step S8; YES) , the operation of the control apparatus 140 proceeds to the new registration process in Step S9. In a case that no new registration of the radiographing order information has been assigned (Step S8; NO), the operation of the control apparatus 140 proceeds to the process in Step S10.

Here, at first, the case that the new registration has been assigned will be described.

With reference to FIG. 9, the new registration process in Step S9 will be described.

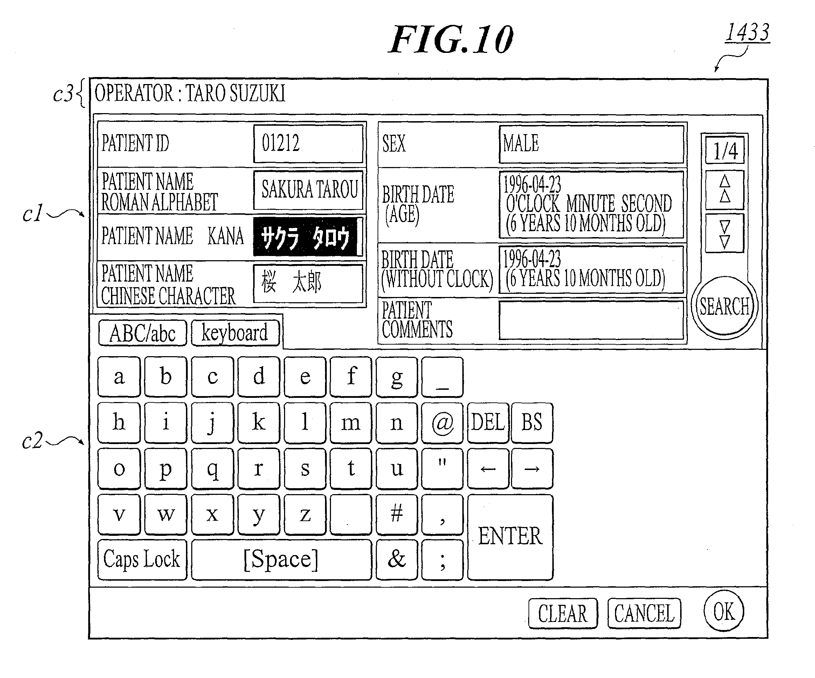

In the new registration process shown in FIG. 9, at first, an input screen 1433 (see FIG. 10) for inputting patient information is displayed in Step S91. As shown in FIG. 10, in the input screen 1433, an input area c1 for inputting various patient information such as a patient ID, a patient name (in Roman alphabet, kana and Chinese Character), a sex, a birth date and the like, and letter keys c2 for inputting letters are displayed. Moreover, the name of the assigned operator is displayed in a display area c3.

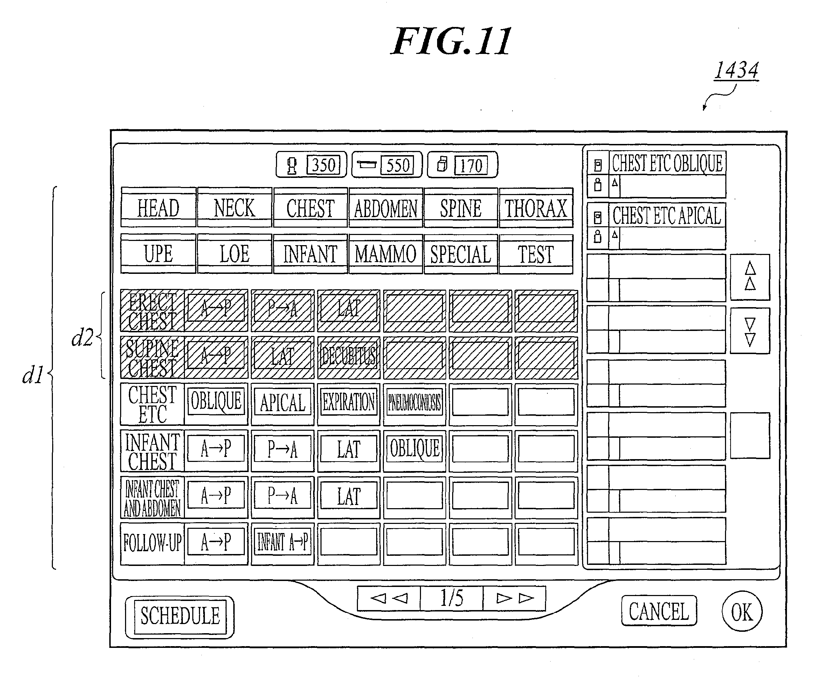

Next, in Step S92, after the operator inputs the patient information to each input area c1 with the letter keys c2, in Step S93, a selection screen 1434 of radiographing conditions (see FIG. 11) for setting a radiographing condition is displayed. As shown in FIG. 11, in the selection screen 1434, a radiographing condition key group d1 for selecting a radiographic part and a radiographing direction is displayed. The radiographing condition key group d1 is a group of keys for selecting the radiographic part and the radiographing direction, which can be radiographed in the normal radiography. Among the keys in the radiographing condition key group d1, keys in a radiographing condition key group d2 for the radiographic parts and radiographing directions incapable of portable radiography are displayed with a mesh for showing that the keys cannot be selected. Here, in the first embodiment, the example that the keys in the radiographing condition key group d2, which cannot be selected, is displayed with a mesh is described. However, the way of displaying the radiographing condition key group d2 is not limited to the one with a mesh, but it may be a way of not displaying the radiographing condition key group d2. Or, the keys may be displayed without a mesh as well as the selectable keys in the radiographing condition key group d1, and when the keys in the radiographing condition key group d2, which cannot be selected, are erroneously selected by an operator, a warning that the keys cannot be selected may be given.

Next, in Step S94, after the operator selects the radiographing condition with the radiographing condition key group d1, new order ID is issued according to the input patient information and the selected radiographing condition, and newly registered to the radiographing order information file 1461 in Step S95. Then, the operator ID of the assigned operator is relatedly stored with the newly registered radiographing order information. Next, in Step S96, the patient list screen 1432 including newly registered radiographing order information is re-displayed, and the operation proceeds to the next process, that is, the process in Step S8 in FIG. 6.

Next, the case that the new registration of radiographing order information has not been assigned in Step S8 in FIG. 6 will be described.

In Step S10, whether or not a patient has been selected for transmitting the radiographing order information thereof to the portable terminal 160 among the patients displayed in a list on the patient list screen 1432 is judged. When the patient is selected for transmitting the radiographing order information thereof (Step S10; YES), the process proceeds to the order transmission process in Step S11. When the patient is not selected for transmitting the radiographing order information thereof (Step S10; NO), the operation returns to Step S8.

With reference to FIG. 12, the order transmission process in Step S11 will be described.

In the order transmission process shown in FIG. 12, the input of a transmission instruction with the transmission key b3 on the patient list screen 1432 is awaited, and whether the transmission instruction has been input or not is judged in Step S111. After the transmission instruction is input (Step S111; YES), whether the portable terminal 160 is attached to the communication terminal 160a or not is judged on the basis of the detection signal of the portable terminal 160 output from the communication terminal 160a (Step S112).

In a case that the detection signal has not been output and the portable terminal 160 is not judged as attached to the communication terminal 160a (Step S112; NO), the attachment of the portable terminal 160 has been awaited for a predetermined period. In a case that the detection signal has been output and the portable terminal 160 is judged as attached to the communication terminal 160a (Step S112; YES) , the radiographing order information of the selected patient is transmitted to the attached portable terminal 160 (Step S113). After the transmission, the transmitted flag of the radiographing order information transmitted to the portable terminal 160 in the radiographing order information file 1461 is set "ON" in Step S114.

Next, in Step S115, a confirmation screen 1435 (see FIG. 13A) for confirming the transmission completion of the radiographing order information is displayed on the display unit 143, and shows that the order transmission process is completed. As shown in FIG. 13A, on the confirmation screen 1435, a transmitting and receiving status b6 is displayed to each patient displayed in a list. As shown in FIG. 13B, a mark "→" indicating that the transmission already has been performed is displayed in the transmitting and receiving status b6 of a patient whose radiographing order information has been transmitted to the portable terminal 160 among the patients displayed in the list. On the other hand, in a case that cassette registration information has been received from the portable terminal 160, a mark "←" indicating that the cassette registration information already has been received is displayed in the transmitting and receiving status b6 of the patient as shown in FIG. 13C.

After the operator confirms that the radiographing order information has been transmitted to the portable terminal 160 on the confirmation screen 1435, the operator conveys the cassette c and the portable radiographing apparatus 110 while carrying the portable terminal 160 to the patient's location of the patient to be radiographed, and prepares radiography.

Next, with reference to FIG. 14, the cassette registration process executed by the portable terminal 160 will be described. The cassette registration process is a process for relating the radiographing order information transmitted from the control apparatus 140 with the cassette c to be used for the radiography.

In the cassette registration process shown in FIG. 14, the reception of radiographing order information from the control apparatus 140 is awaited. In Step P1, whether the radiographing order information has been received or not from the control apparatus 140 via the communication terminal 160a is judged.

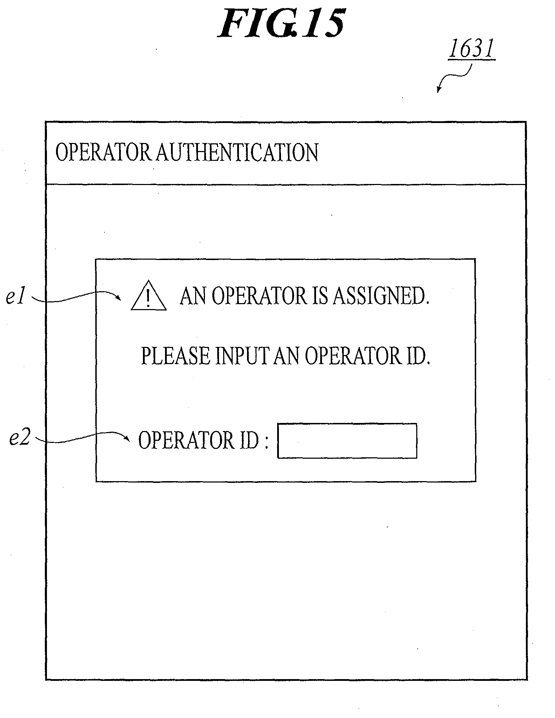

When the portable terminal 160 receives the radiographing order information from the control apparatus 140 (Step P1; YES), the received radiographing order information is stored in the radiographing order information file 1661 (Step P2), and an authentication screen 1631 (see FIG. 15) to authenticate an operator is displayed on the display unit 163 in Step P3. As shown in FIG. 15, on the authentication screen 1631, an input area e2 for inputting the operator ID is displayed along with a message e1 informing that an operator is assigned, and guidance for inputting the operator ID is given.

When the operator ID is input on the authentication screen 1631 by the operator carrying the portable terminal 160, it is judged in Step P4 whether the input operator ID corresponds to the operator ID of the operator assigned by the radiographing order information (Step P4). When the input operator ID does not correspond to the assigned operator ID (Step P4; NO), a warning message for warning that the input operator ID does not correspond to the assigned operator is displayed on the display unit 163, or a warning sound is output (Step P5).

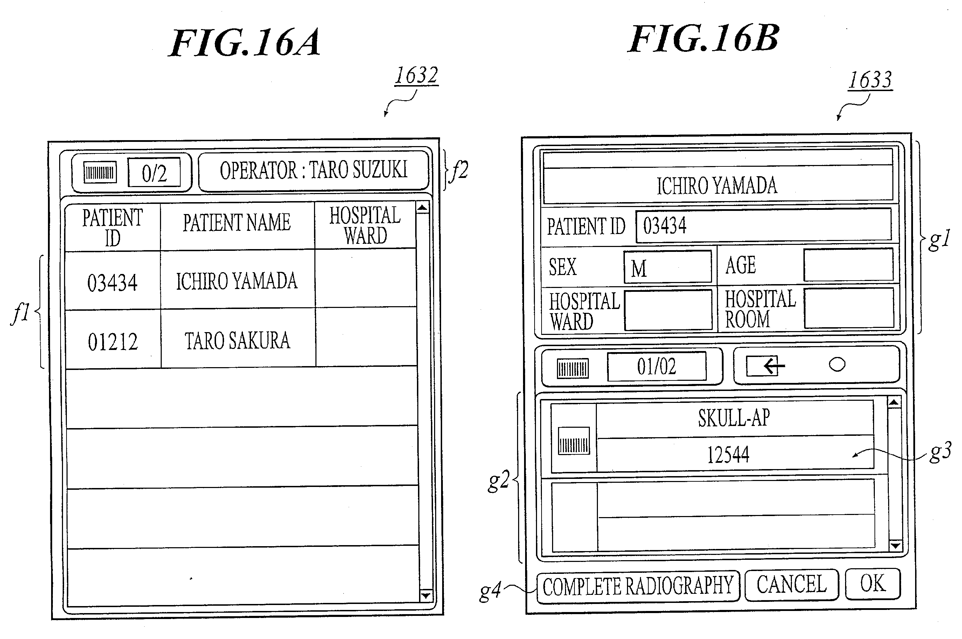

On the other hand, when the input operator ID corresponds to the assigned operator ID (Step P4; YES), a patient list screen 1632 (see FIG. 16A) is displayed on the display unit 163 on the basis of the radiographing order information stored in the radiographing order information file 1661 (Step P6). As shown in FIG. 16A, on the patient list screen 1632, a patient ID, a patient name and the like of a patient to be radiographed is displayed as a list in a display area f1, and the name of the assigned operator is displayed in a display area f2. Here, a situation where the radiographing order information having too much information to display on the display area f1 at once, is compensated by scrolling the screen in order display all the information. The operator confirms the patient list screen 1632 displayed on the portable terminal 160 at a bedside of a patient to be radiographed, for selecting and inputting the patient to be radiographed among the patients displayed in the list.

When the patient to be radiographed is selected by the operator among the patients displayed in the list on the patient list screen 1632, an order list screen 1633 (see FIG. 16B) is displayed, and a radiographing condition g2 and the patient information g1 of the selected patient is displayed in a list in Step P7.

With a cursor, the operator assigns the radiographing condition g2 in the radiographing order information which the operator wants to relate with a cassette among the radiographing conditions g2 displayed in the list on the order list screen 1633. Then, the operator has the portable terminal 160 read the barcode of the cassette ID of the cassette c to be used for radiography with the barcode reader 167 of the portable terminal 160.

The portable terminal 160 awaits the assignation of the radiographing order information to be registered on the order list screen 1633. When the operator assigns the radiographing condition g2 of the radiographing order information to be registered (Step P8; YES), the barcode reader 167 reads the barcode of the cassette ID, and whether the cassette ID has been obtained or not is judged (Step P9) . In a case that the cassette ID has not been obtained (Step P9; NO), the portable terminal 160 awaits to obtain the cassette ID.

In a case that the cassette ID has been obtained (Step P9; YES), the assigned radiographing order information and the obtained cassette ID are related with each other and are stored in the cassette registration information file 1662 (Step P10). When the registration is completed, the cassette ID g3 of the cassette c registered in the radiographing condition g2 is displayed in the order list screen 1633 as shown in FIG. 16B, and the cassette registration is informed. Moreover, at the lower part of the screen, a radiography completion key g4 for inputting radiography completion information for informing radiography completion is displayed.

The operator confirms the completion of the cassette registration on the order list screen 1633, and performs the radiography of a patient with the registered cassette c. Here, it is possible that, when a plurality of patients are continuously radiographed at a patient's location, the processes from Step P2 to Step P7 are repeated, and each cassette registration information is successively stored in the cassette registration information file 1662. Moreover, regardless of the turn of the cassette registration and the radiography, the portable terminal 160 may perform the cassette registration after the radiography. Then, at the completion of the radiography, the operator pushes down the radiography completion key g4 in the order list screen 1633, and the operator returns to the radiography room where the control apparatus 140 is installed. Then, the operator attaches the portable terminal 160 to the communication terminal 160a connected to the control apparatus 140.

In Step P11, the portable terminal 160 judges whether the portable terminal 160 is attached to the communication terminal 160a or not. In a case that it is judged that the portable terminal 160 is not attached to the communication terminal 160a (Step P11; NO) , attaching the portable terminal 160 is awaited. On the other hand, in a case that it is judged that the portable terminal 160 is attached to the communication terminal 160a (Step P11; YES), the cassette registration information is transmitted to the control apparatus 140 connected to the portable terminal 160 via the communication terminal 160a (Step P12).

Next, in Step P13, after the radiographing order information is selected, whether the radiography completion key g4 is pushed down or not is judged. In a case that the radiography completion key g4 has not been pushed down (Step P13; NO), the cassette registration process is terminated. In a case that the radiography completion key g4 has been pushed down (Step P13; YES), the radiographed flag of the selected radiographing order information is set "ON" in the radiographing order information file 1661 (Step P14). Successively, in Step P15, the order ID of the radiographing order information having the radiographed flag "ON" is transmitted to the control apparatus 140, and the cassette registration process is completed.

Then, the operator attaches the portable terminal 160 to the communication terminal 160a when the radiography is completed and the operator returns to the radiography room where the control apparatus 140 is installed. Here, in case the medical image radiographing system 100 does not comprise the radiography completion key g4, the medical image radiographing system 100 judges that the radiography of the radiographing order information is completed when the portable terminal 160 is attached to the communication terminal 160a, then sets the radiographed flag of the radiographing order information related to the cassette "ON" automatically, and further transmits the order ID of the radiographing order information having the radiographed flag "ON" to the control apparatus 140 along with the cassette registration information.

After the operator attaches the portable terminal 160 to the communication terminal 160a and transmits the cassette registration information to the control apparatus 140, the operator attaches the cassette c to the reading apparatus 120 and operates the control apparatus 140 to read the medical image recorded in the cassette c. The reading apparatus 120 reads the barcode of the cassette ID from the attached cassette c, as well as the medical image. Then, the cassette ID of the cassette c of the medical image is written in a header area of the read medical image. The medical image with the cassette ID written therein is transmitted to the control apparatus 140.

Next, the image registration process executed by the control apparatus 140 will be described with reference to FIG. 17. The image registration process is the process for relating the radiographing order information to the radiography image transmitted from the reading apparatus 120 on the basis of the cassette registration information transmitted from the portable terminal 160.

In the image registration process shown in FIG. 17, in Step T1, it is judged whether the cassette registration information is received from the portable terminal 160 through the communication terminal 160a or not. In a case that the cassette registration information has not been received (Step T1; NO), the control apparatus 140 awaits the reception. In a case that the cassette registration information has been received (Step T1; YES), the radiographing order information and the cassette ID of the cassette c used for the corresponding radiography are related to each other on the basis of the received cassette registration information, and stored in the registered information file 1462 (Step T2).

Next, in Step T3, the confirmation screen 1435 (see FIG. 13A) is displayed on the display unit 143 for confirming the reception of the cassette registration information, which corresponds to the radiographing order information transmitted from the control apparatus 140 to the portable terminal 160, from the portable terminal 160. As shown in FIG. 13A, the mark "←" indicating that the cassette registration information to the radiographing order information has been received is displayed in the transmitting and receiving status b6 on the confirmation screen 1435. After the confirmation screen 1435 is displayed, the image registration process proceeds to the radiographed order process in Step T4.

With reference to FIG. 18, the radiographed order process in Step T4 will be described.

In the radiographed order process shown in FIG. 18, at first, in Step T41, the reception of the order ID of the radiographing order information of which radiography has completed from the portable terminal 160 is awaited, and it is judged whether the order ID has been received or not. In a case that the order ID of the radiography-completed radiographing order information has been received (Step T41; YES), the radiographed flag of the radiographing order information of the received order ID is set "ON" in the radiographing order information file 1461 (Step T42).

Next, in Step T43, the order ID of the radiography-completed radiographing order information having the radiographed flag "ON" is transmitted to another control apparatus 140 by the communication unit 144 through the communication network N. Next, in Step T44, it is judged whether the order ID of the radiography-completed radiographing order information has been received from another control apparatus through the communication unit 144 or not. In a case that the radiographing order information has been received (Step T44; YES), the radiographing order information corresponding to the received order ID is deleted from the radiographing order information file 1461 (Step T45) , and the radiographed order process proceeds to the next process, i.e. Step T5 in FIG. 17. On the other hand, in a case that the order ID of the radiography-completed radiographing order information has not been received from another control apparatus (Step T45; NO) , the process in Step T45 is not performed, and the radiographed order process goes to the process in Step T5 in FIG. 17.

In Step T5 in FIG. 17, it is judged whether the read medical image has been received from the reading apparatus 120 or not. In a case that the medical image has not been received (Step T5; NO), the reception of the medical image is awaited. In a case that the medical image has been received (Step T5; YES), cassette ID is read from the header area of the received medical image, and a file name of the medical image is stored in the registered information file 1462 related to the cassette ID corresponding to the read cassette ID (Step T6).

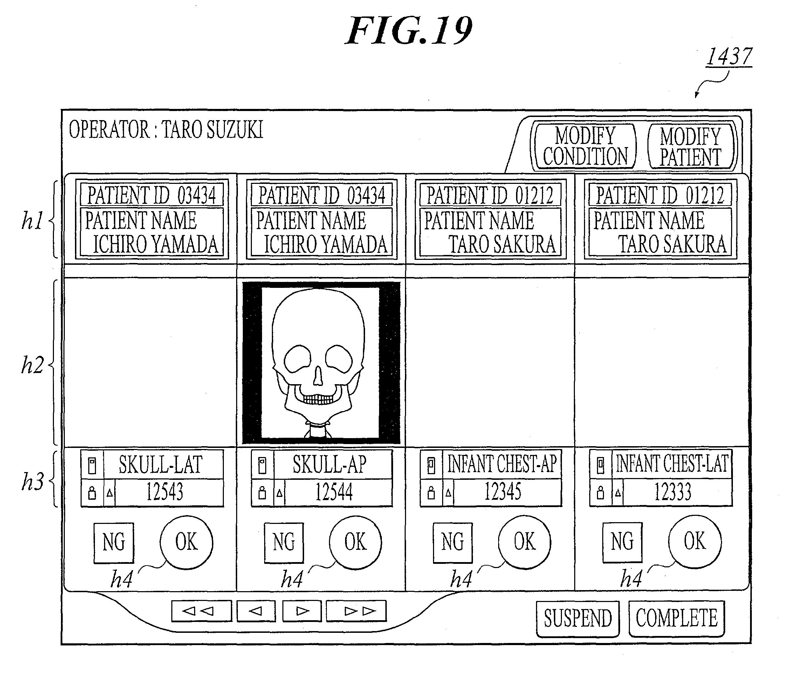

Next, in Step T7, the display unit 143 displays an image confirmation screen 1437 (see FIG. 19) showing the radiographing order information and the medical image radiographed according thereto are displayed relatedly to each other. As shown in FIG. 19, in the image confirmation screen 1437, medical images h2 of a plurality of patients are displayed parallelly. In each of the medical images h2, patient information h1 including a patient ID and a patient name, and radiographing information h3 including radiographing conditions and the cassette ID of a cassette used for the radiography are relatedly displayed. Here, in a case that cassette registration has been performed but no medical image has been read, only the patient information h1 and the radiographing information h3 are displayed, and no image is displayed in h2. The operator confirms the correspondence relation between the displayed medical image and the radiographing order information, and when they correspond to each other, the operator pushes down an OK key h4.

When the OK key h4 is pushed down in the image confirmation screen 1437, the data file of the medical image corresponding to the pushed OK key h4 is transmitted to the information management apparatus 130, and the correspondence relation between the medical image and the radiographing order information is read from the registered information file 1462 to be transmitted to the information management apparatus 130 as the image registration information in Step T8. Then, the radiographed order process is completed.

Then, the control apparatus 140 sends the medical image to an image database (not shown) and the information management apparatus 130 receives the image registration information from the control apparatus 140. Finally, the medical image is managed in the database on the basis of the received image registration information.

As described above, in the medical image radiographing system 100, the plurality of portable terminals 160 are connected to the specific control apparatus 140 via the communication terminals 160a, and radiographing order information for portable radiography is transmitted from the control apparatus 140 to the portable terminals 160 to be displayed thereon. As a result, even when radiography is performed at a patient's location such as a bedside of a patient or the like, an operator can confirm radiographing order information with a carried portable terminal 160, and thereby the operator can easily confirm the patient to be radiographed and the radiographing conditions. Consequently, the radiography of a medical image at the patient's location can be performed efficiently and accurately.

Moreover, when the portable terminal 160 reads the cassette ID to be used for radiography and cassette registration is performed and further the cassette registration information is transmitted from the portable terminal 160 to the control apparatus 140, the control apparatus 140 relates the radiographed medical image to the radiographing order information on the basis of the cassette registration information. As a result, it is possible to omit the operator's operation of inputting the correspondence relation between the radiographing order information and the medical images in the control apparatus 140, and thereby possible to reduce the operation labor. Moreover, human errors caused by input mistakes of an operator can be prevented, and medical images can be accurately managed.

Moreover, when the control apparatus 140 assigns an operator who performs radiography, the operator ID of the assigned operator is transmitted to the portable terminal 160 along with the radiographing order information, and the portable terminal 160 authenticates the operator who performs radiography on the basis of the transmitted operator ID. Then, only when the operator is authenticated, the radiographing order information is displayed. As a result, even when identical radiographing order information is redundantly delivered to a plurality of portable terminals 160, only the assigned operator can confirm the radiographing order information. Hence, it is possible to prevent that a plurality of operators simultaneously perform radiography preparations to one patient, or redundantly perform radiography based on the identical radiographing order information. Consequently, the radiography of a medical image can be accurately performed at the patient' s location.

Moreover, when the portable terminal 160 does not authenticate an operator, a warning that the operator is not the assigned operator is given. Consequently, it is possible to draw the operator's attention to the assignation of an operator.

Moreover, when radiography-completed radiographing order information is transmitted to the control apparatus 140 from another control apparatus 140, the radiography-completed radiographing order information is deleted from the radiographing order information file 1461. Moreover, when one control apparatus 140 transmits radiographing order information to one portable terminal 160, the radiographing order information is deleted on each display of the other control apparatuses 140. Consequently, the redundancy of delivering identical radiographing order information to the portable terminals 160 from each control apparatus 140 can be removed and thereby the process efficiency is improved. Moreover, the reduction of the amount of data stored in the storage 146 can be achieved.

Moreover, since the portable terminals 160 are directly connected to the specific control apparatus 140 via the communication terminals 160a in the above-mentioned system structure, it is only necessary to do the maintenance on only the specific control apparatus 140 and the portable terminal 160 connectable to the specific control apparatus 140 at the time of a system maintenance or a system change. Therefore, the system can be changed more flexibly and therefore it is easy to rebuild the system. Moreover, by specifying the control apparatus 140 to which portable terminal 160 is connected, the stabilization of the system can be achieved.

Here, the description related to the first embodiment is a suitable example of the medical image radiographing system 100 to which the present invention is applied, and the present invention is not limited to the description.

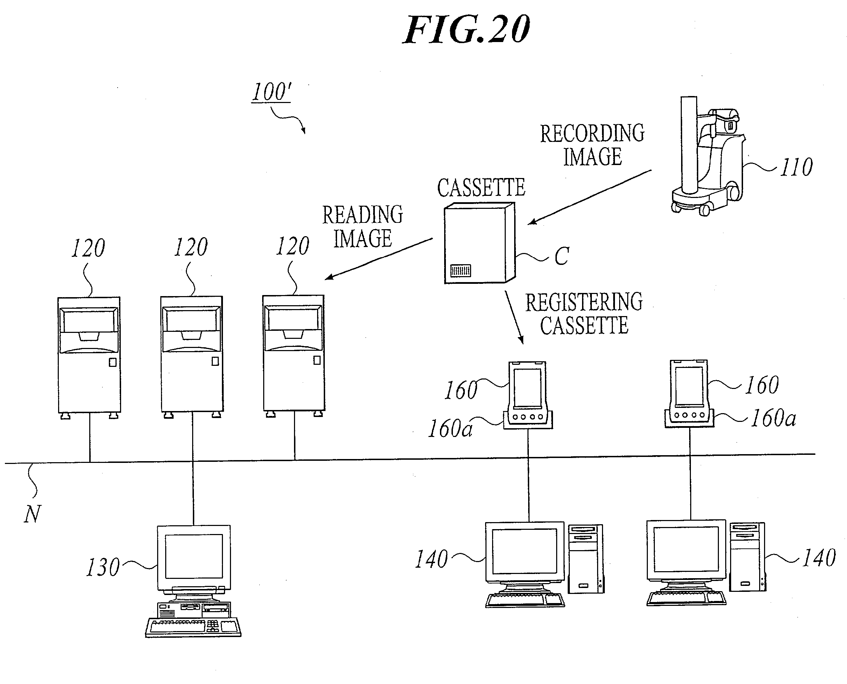

In the above description, the system structure wherein the portable terminal 160 is connected to the specific control apparatus 140 is used. However, as a medical image radiographing system 100' shown in FIG. 20, the portable terminal 160 may be connected to the communication network N directly via the communication terminals 160a, and the portable terminals 160 can communicate with any control apparatus 140 through the communication network N.

In the structure of the medical image radiographing system 100' shown in FIG. 20, when the control apparatus 140 transmits radiographing order information to the portable terminal 160, while one among all the control apparatuses is assigned as a transmitting target of cassette registration information corresponding to the radiographing order information, the control apparatus 140 also transmits information (for example, IP address or the like) of the assigned control apparatus accompanied with the radiographing order information as accompanying information. Here, when the control apparatus transmitting cassette registration information is not specifically assigned, the cassette registration information is transmitted from the portable terminal 160 to the control apparatus which is an original transmitter of the radiographing order information.

Since the portable terminals 160 can be connected to any of the control apparatus 140 in the system structure of the medical image radiographing system 100', any control apparatus 140 can transmit the radiographing order information to a desired portable terminal 160, and consequently user-friendliness is improved.

Besides, the detail of the structure and the operation of the medical image radiographing system 100 of the first embodiment may be suitably changed without departing from the essence of the present invention.

### [Second Embodiment]

Next, as a second embodiment, a medical image radiographing system wherein portable terminals are directly connected to a network and capable of transmitting and receiving data with information management apparatus and control apparatuses to each other through the network, such as the above-mentioned medical image radiographing system 100' shown in FIG. 20, will be described further in detail.

Hereinafter, with reference to the figures, the second embodiment of the present invention will be described in detail.

In the second embodiment, as one form of the portable terminal disclosed in the claims, an example where the present invention is applied to a portable information terminal (hereinafter referred to as PDA (Personal Digital Assistant) ) 210 will be described.

First, the structure of the embodiment will be described.

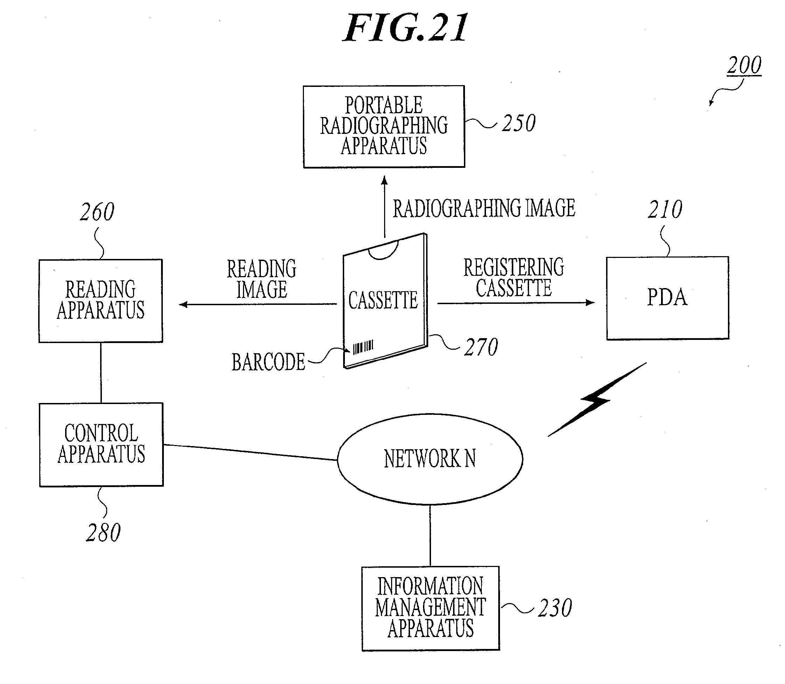

FIG. 21 is a conceptual diagram showing a whole structure of a medical image radiographing system 200 including the PDA 210 according to the present invention.

In FIG. 21, the medical image radiographing system 200 comprises the PDA 210, a portable radiographing apparatus 250, a medical image reading apparatus 260 for cassette use, a cassette 270 and a control apparatus 280. The PDA 210, an information management apparatus 230 and the control apparatus 280 are connected through a network N and capable of transmitting and receiving data to each other.

The network N can take various line forms such as a local area network (LAN), a wide area network (WAN), the Internet and the like. Here, the network N may take radio communication or infrared-ray communication if it is allowed in a medical institution such as a hospital and the like. Since the radiographing order information includes important patient information, while it is transmitted and received, the radiographing order information is encoded.

The information management apparatus 230 stores radiographing order information input with operations of a doctor, and transmits the radiographing order information to the PDA 210 by way of the control apparatus 280 or directly, the control apparatus 280, and the medical image reading apparatus 260, which are connected to the information management apparatus 230 through the network N. Here, as another information management apparatus, an entry apparatus for entering reservation of radiographing order information may be used, or an information management system such as the HIS, the RIS or the like may be used.

The PDA 210 is a portable radiographing information apparatus carried by an operator such as a radiologic technologist. The PDA 210 receives, by way of the control apparatus 280 or directly, the radiographing order information from the information management apparatus 230 and stores the received radiographing order information therein. The PDA 210 displays assigned radiographing order information at the time of radiography, and registers the cassette 270 to be used for the radiography according to each radiographing order information so as to identify the cassette 270 from the others.

The portable radiographing apparatus 250, which is movable, radiographs a patient at a patient's location in accordance with an instruction of the operator, and records the radiographed medical image in the cassette 270.

The medical image reading apparatus 260, a medical image reading apparatus for cassette use exclusively, reads the medical image recorded in the cassette 270. The control apparatus 280 controls the reading operation of the medical image reading apparatus 260, and relates the radiographing order information received from the information management apparatus 230 with the medical image read out of the cassette 270 for image management.

Next, the PDA 210 according to the present invention will be described in detail.

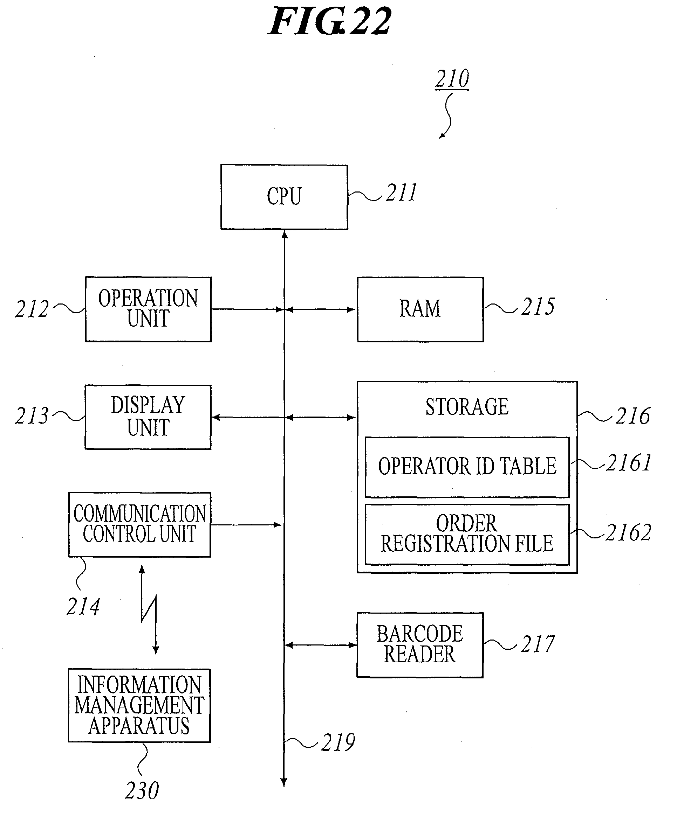

FIG. 22 is a block diagram showing a functional structure of the PDA 210 in the second embodiment. In FIG. 22, the PDA 210 comprises a CPU (Central Processing Unit) 211, an operation unit 212, a display unit 213, a communication control unit 214, a RAM (Random Access Memory) 215, a storage 216 and a barcode reader 217. Each unit is connected to each other through a bus 219.

The CPU 211 develops a system program stored in the storage 216 and an assigned program among various application programs into the RAM 215, and integrally controls each unit of the PDA 210 in accordance with the programs.

Concretely, the CPU 211 achieves an identification information authentication section and a display control section by reading an order authentication process program from the storage 216.

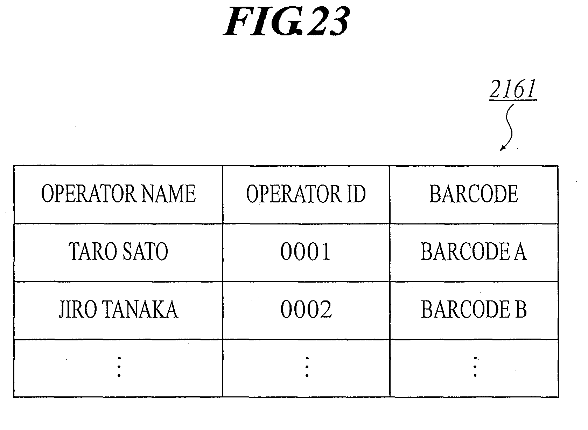

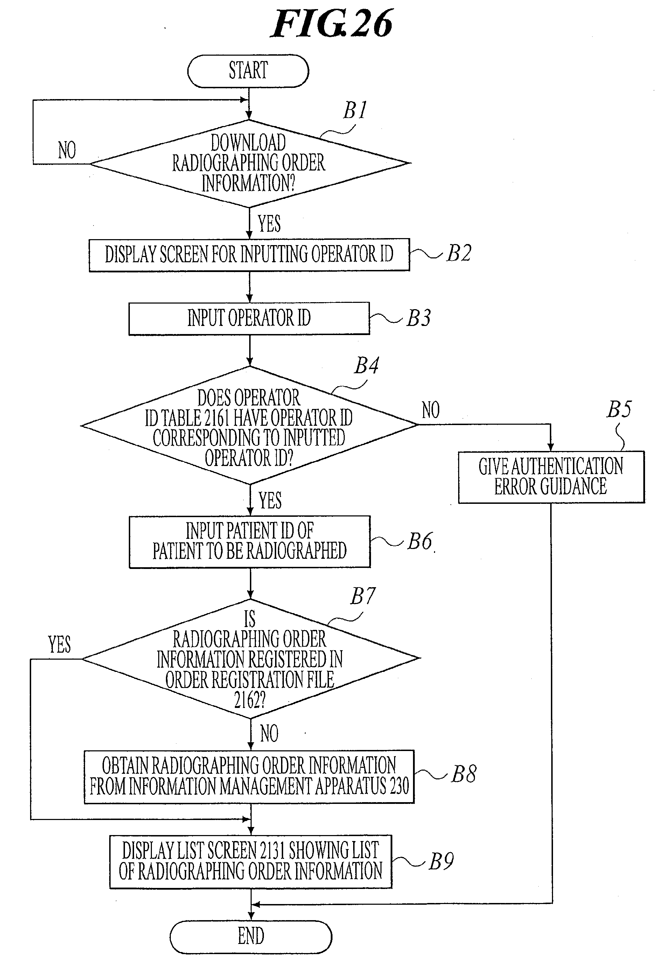

When an instruction of displaying the radiographing order information is input to the CPU 211 through the operation unit 212 in an order authentication process (see FIG. 26), the CPU 211 displays an input screen for inputting an operator ID as operator identification information on the display unit 213. When the operator ID is input on the input screen, the CPU 211 refers to an operator ID table 2161 (see FIG. 23) in the storage 216 to judge whether an operator ID corresponding to the input operator ID is registered or not. When no operator ID corresponding to the input operator ID is registered, the CPU 211 does not display any radiographing order information, and gives authentication error guidance for informing that the operator ID is not authenticated.

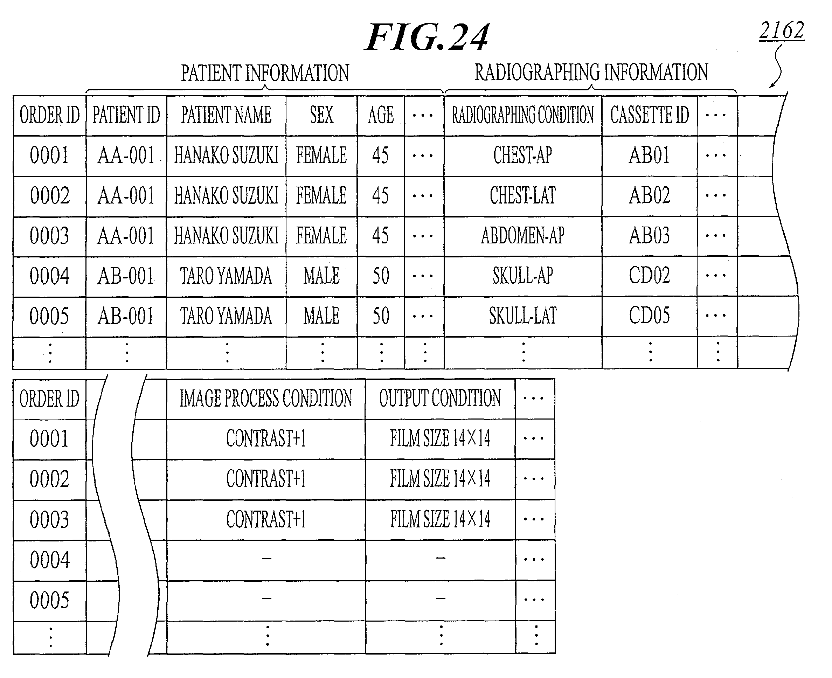

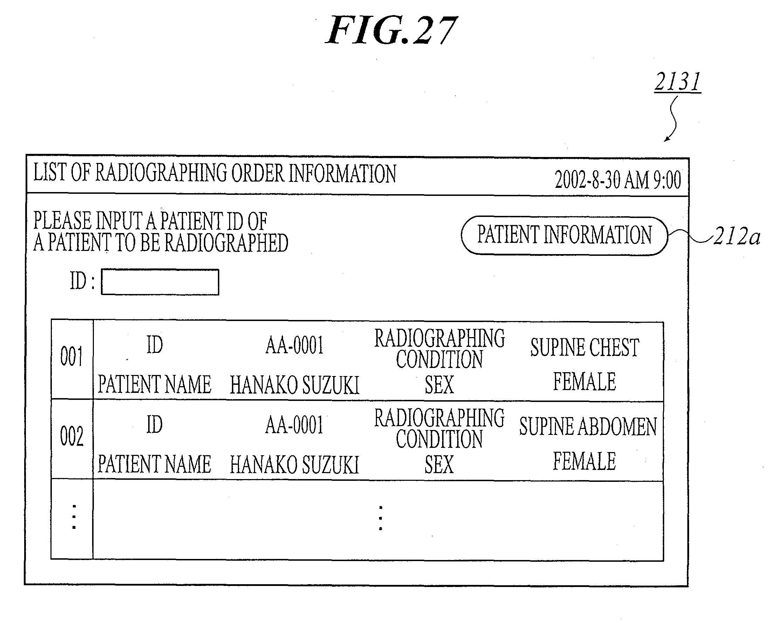

On the other hand, when an operator ID corresponding to the input operator ID is registered, the CPU 211 displays an input screen for inputting a patient ID of a patient to be radiographed on the display unit 213, and judges whether radiographing order information corresponding to the input patient ID is registered in an order registration file 2162 (see FIG. 24) in the storage 216 or not. When the corresponding radiographing order information is registered in the order registration file 2162, the CPU 211 downloads the radiographing order information from the order registration file 2162, and displays a radiographing order information list screen 2131 (see FIG. 27) for showing the downloaded radiographing order information as a list on the display unit 213. When the corresponding radiographing order information is not registered in the order registration file 2162, the CPU 211 obtains the radiographing order information corresponding to the patient ID from the information management apparatus 230 through the communication control unit 214 and displays the list screen 2131 of the obtained radiographing order information.

The operation unit 212, one of input sections for inputting operator ID, comprises cursor keys, numeric keys and various function keys. The operation unit 212 receives the operator ID input by an operator according to key operations, and outputs the input operator ID to the CPU 211. Here, the operation unit 212 may comprise a pointing device such as a mouse, a touch panel or the like, and other operation input devices.

The display unit 213 is a display section provided with a display using a liquid crystal display (LCD) or the like. The display unit 213 displays various information such as radiographing order information, patient information and the like on the basis of display instructions from the CPU 211.

The communication control unit 214, a receiving section, comprises a network interface card, a modem, a terminal adapter or the like. The communication control unit 214 controls communication with external devices through the network N. For example, the communication control unit 214 establishes radio communication with the information management apparatus 230, and receives radiographing order information through the network N. Here, in this case, if necessary, the radio communication may be established with a portable telephone terminal such as the PHS or the like.

The RAM 215 forms a work area for temporarily storing various programs to be executed by the CPU 211 and data processed by the various programs.

The storage 216 comprises a storage medium (not shown) in which programs and data are previously stored. The storage medium stores a system program, various application programs corresponding to the system program, data processed by the various process programs and the like. Moreover, the storage medium is composed of a magnetic storage medium, an optical storage medium or a semiconductor memory. The storage medium is provided in the storage 216, either fixedly or detachably.

Moreover, the storage 216 has an operator ID table 2161 therein for storing the operator identification information. As a data storage example of the operator ID table 2161 shown in FIG. 23, the operator ID table 2161 stores operator names, which are previously registered, operator IDs as operator identification information, and barcodes which are coded from operator IDs. For example, as shown in FIG. 23, the operator ID table 2161 stores the operator ID of the operator "TARO SATO" as "0001", and the barcode of the operator as "BARCODE A".

Moreover, the storage 216 comprises the order registration file 2162. The order registration file 2162 is a radiographing order information storage section for storing the radiographing order information received from the information management apparatus 230 through the communication control unit 214.

Concretely, as a data storage example of the order registration file 2162 shown in FIG. 24, the order registration file 2162 stores, in the turn of the order ID, patient information such as a patient ID of a patient to be radiographed (for example "AA-001"), a patient name (for example "HANAKO SUZUKI"), a sex (for example "FEMALE", "MALE"), age (for example "45") and the like; radiographing information such as a radiographing condition (including a radiographic part, a radiographing direction; for example "CHEST AP"), cassette ID as a identification number of a cassette used for radiography (for example "AB01") and the like; an image process condition of radiographed medical images (for example "CONTRAST+1", which indicates to raise a contrast level by one in a gradation process) ; and an output condition of the radiographed medical images (for example "FILM SIZE 14×14", which indicates a film size at the time of outputting as a film).

The patient information includes, in addition to a patient ID, a name, a sex, age of a patient, which are shown in FIG. 24, various types of information, such as a hospital department requesting the examination, a doctor in charge, a hospital room name, medical warning information for warning an infectious disease and the like, drug allergy, pregnancy, additional medical history, special care necessity such as a wheelchair, a stretcher or the like, a clinical diagnosis name, a secret matter and the like. Moreover, the radiographing information includes, in addition to the radiographing conditions and the cassette ID used for radiography, which are shown in FIG. 24, various types of information such as a radiographing method (simple radiography, contrast radiography or the like), a radiographing date, a radiographing apparatus, a reading apparatus and the like.

Further, the image process condition includes, in addition to the image process condition upon a gradation process shown in FIG. 24, image process conditions upon various types of processes such as a frequency process for adjusting the sharpness of images, a dynamic range compressing process for converging data of an image having wide dynamic range within a density range in which the image can be easily observed without decreasing the contrast of the details of a subject, a compression process for compressing image data in accordance with a predetermined compression method, and the like. Moreover, the output condition includes, in addition to a film size at the time of outputting the film shown in FIG. 24, output conditions of various outputting methods such as inversion, rotation, brightness, contrast and the like at the time of displaying a radiographed image.

The barcode reader 217, one of input sections for inputting an operator ID, includes a scanner as an optical reading mechanism. The barcode reader 217 reads the barcode of operator ID, and outputs the barcode data to the CPU 211.

Next, an operation of the second embodiment will be described.

The programs for achieving each function described in flowcharts described later are stored in the storage 216 of the PDA 210 as a form of program codes capable of being read by a computer. The CPU 211 of the PDA 210 sequentially executes the operation in accordance with the program codes.

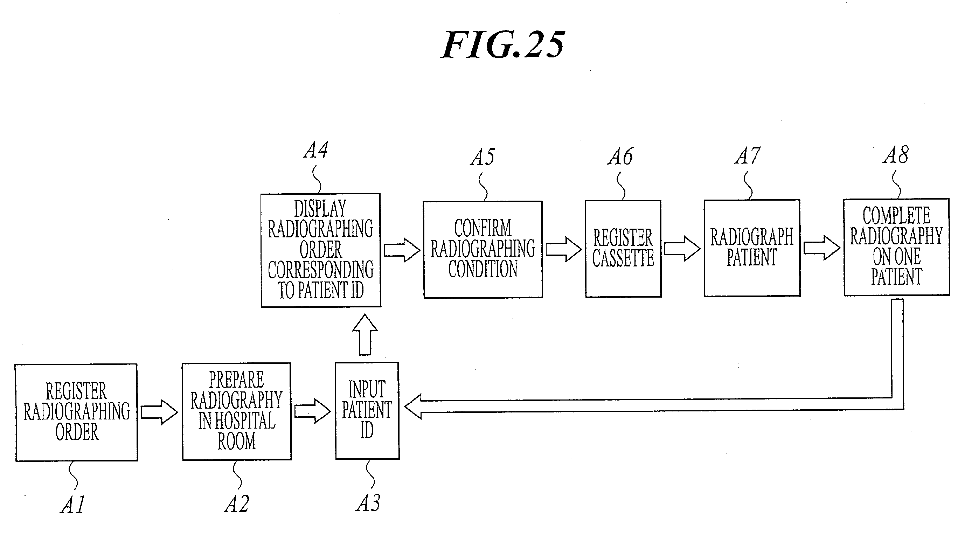

With reference to FIG. 25, at first, an overall radiography flow of the medical image radiographing system 200 using the PDA 210 will be described. The PDA 210 is used at the time of radiography of a patient at a hospital room or the like where the patient stays, with the portable radiographing apparatus 250 and the cassette 270, the patient incapable of traveling to a radiography room due to, for example, the difficulty of walking, being in an operation, or the like.

In FIG. 25, the information management apparatus 230 first transmits the radiographing order information to the corresponding PDA 210. The PDA 210 stores the radiographing order information received from the information management apparatus 230 in the order registration file 2162, and registers the radiographing order information (Step A1).

Next, an operator conveys the portable radiographing apparatus 250 and the cassette 270 to the hospital room where the patient stays, and starts radiography preparation (Step A2). When the radiography is ready, the operator operates the PDA 210 to instruct the display of the radiographing order information. The PDA 210 executes the order authentication process, which will be described later, in accordance with the instruction of displaying the radiographing order information. When the operator is authenticated, the operator inputs the patient ID of the patient to be radiographed in the PDA 210 (Step A3), and the PDA 210 downloads the radiographing order information corresponding to the input patient ID from the order registration file 2162, and displays the radiographing order information as a list (Step A4).

When the operator refers to the radiographing order information displayed on the PDA 210 and confirms the radiographic condition (Step A5), the PDA 210 registers the cassette to be used for radiography to the radiographing order information (Step A6). Then, the operator radiographs the patient with the registered cassette 270 and the portable radiographing apparatus 250 on the basis of the radiographing order information displayed on the PDA 210 (Step A7). In this way, the radiography is performed and when the radiography is completed to the patient on the basis of one or a plurality of radiographing order information (Step A8), the operator inputs the patient ID of the patient to be radiographed next in the PDA 210 (Step A4), and equally performs a series of radiography to the next patient.

The operator inserts the cassette 270 wherein the radiography image is recorded into the medical image reading apparatus 260 after the series of radiography for reading the radiographed image. The control apparatus 280 stores previously received radiographing order information from the information management apparatus 230 therein. Then, the control apparatus 280 relates the radiography image read by the medical image reading apparatus 260 with the radiographing order information assigned by the operator to store them.

Next, with reference to a flowchart in FIG. 26, the order authentication process executed by the CPU 211, when the PDA 210 displays the radiographing order information of the series of radiography, as described above will be described.

In FIG. 26, when the CPU 211 receives the input of instruction of displaying radiographing order information from the operation unit 212 (Step B1; YES), the CPU 211 displays an input screen on the display unit 213 (Step B2) for an operator to input an operator ID.

Next, the operator inputs the operator ID at the operation unit 212. Or, when reading of the operator ID in a barcode is instructed, the barcode reader 217 reads the barcode to input it. When the CPU 211 obtains the operator ID from the barcode (Step B3), the CPU 211 downloads the operator ID table 2161, and judges whether an operator ID corresponding to the input operator ID is registered or not (Step B4).

When no operator ID corresponding to the input operator ID is registered, the CPU 211 displays an message such as "OPERATOR AUTHENTICATION FAILED", "OPERATOR ID IS WRONG" or the like on the display unit 213 to give authentication error guidance. Moreover, the CPU 211 does not display any radiographing order information on the display unit 213 (Step B5), and completes the order information authentication process.

On the other hand, when an operator ID corresponding to the input operator ID is registered in the operator ID table 2161, the CPU 211 authenticates the operator ID, and has the display unit 213 display an input screen for inputting a patient ID of a patient to be radiographed. When the patient ID is input on the input screen of the patient ID at the operation unit 212 (Step B6), the CPU 211 judges whether the radiographing order information corresponding to the input patient ID is registered in the order registration file 2162 or not (Step B7).

When the radiographing order information is registered, the CPU 211 downloads the radiographing order information corresponding to the patient ID from the order registration file 2162, and makes the display unit 213 display the list screen 2131 of the radiographing order information as shown in FIG. 27 (Step B9). On the other hand, when no radiographing order information is registered, the CPU 211 obtains the radiographing order information corresponding to the PDA 210 from the information management apparatus 230 through the communication control unit 214 (Step B8), and stores the obtained radiographing order information in the order registration file 2162 to register it. Then, the CPU 211 downloads the radiographing order information corresponding to the patient ID among the registered radiographing order information, and equally displays the list screen 2131 of the radiographing order information (Step B9). Then, the CPU 211 completes the order information authentication process.

With reference to FIG. 27, the list screen 2131 of the radiographing order information will be described in detail.

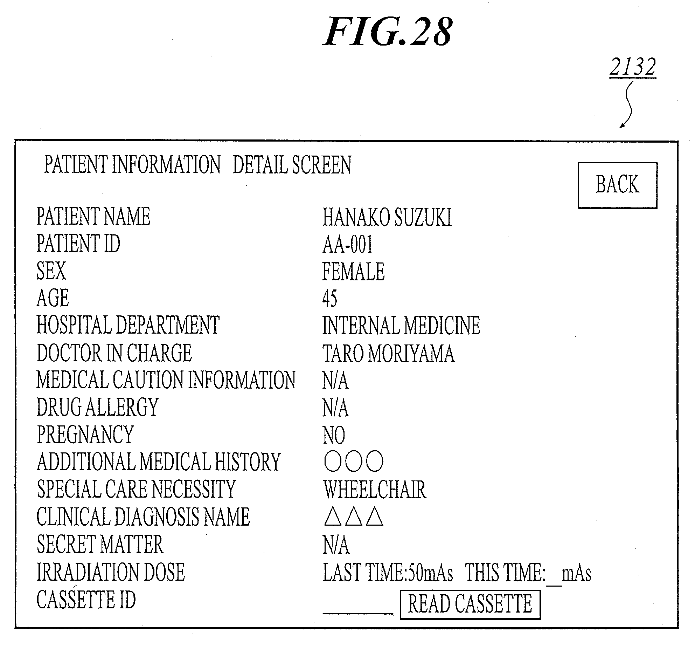

The CPU 211 allocates an area for inputting a patient ID of a patient to be radiographed in the upper part of the radiographing order information list screen 2131 in order to search radiographing order information, and displays a list of the radiographing order information corresponding to the input patient ID in the lower part of the area. Moreover, the CPU 211 allocates a patient information button 212a on the upper right side of the radiographing order information list screen 2131. When the patient information button 212a is pushed down, the CPU 211 shifts the display of the radiographing order information list screen 2131 to a detail screen 2132 for showing further detailed patient information as shown in FIG. 28.

On the detail screen 2132 of FIG. 28, further detailed patient information is displayed. In addition to minimum required patient information for identifying a patient to be radiographed such as a patient name, a sex and the like, which are displayed on the radiographing order information list screen 2131, the detailed screen 2132 displays the information deeply related to privacy of a patient such as medical warning information, drug allergy and the like. Moreover, the radiographing order information list screen 2131 displays a reading button for instructing to read the history of the irradiation dose to a patient, and to read a cassette ID.

As described above, since a plurality of radiographing order information is stored in the PDA 210 carried by the operator and displayed therein, handling of the radiographing order information becomes easy and desired radiographing order information can be easily confirmed. Consequently, labor of an operator for radiography operation can be reduced.

Moreover, when the radiographing order information stored in the PDA 210 is displayed on the display unit 213, operator ID is input, and then, the display of radiographing order information and patient information is controlled according to a result of the authentication of the operator ID, the result in which, when the input operator ID is not authenticated, the radiographing order information and the patient information are not displayed, and when the input operator ID is authenticated, the radiographing order information and the patient information are displayed. Therefore, leakage of the patient information from the PDA 210 to the outside can be prevented. Moreover, an identity of an operator can be easily specified with an operator ID, and therefore security of the system is improved. In particular, since the PDA 210 is portable, there is a high possibility of the leakage of information to the outside. Therefore, it is very effective as a security measure to control the display of the radiographing order information and the patient information after the authentication of an operator.

Moreover, since it is possible to access the information management apparatus 230 to obtain radiographing order information only when the operator ID is authenticated, the security of the information management system such as the RIS, the HIS and the like can be ensured.

Here, the description of the second embodiment is an example suitable for the PDA 210 to which the PDA 210 of the present invention is applied, and the PDA 210 is not limited to the description.

For example, in the second embodiment, the PDA is applied to be described as the portable terminal of the present invention, but, as the portable terminal, a portable notebook personal computer, a portable telephone terminal or the like may be applied.

Moreover, in the above description, an ID number or a barcode of the ID number, which is peculiar to each operator, is applied as operator identification information. However, the operator identification information.is not limited to the ID number and the barcode as long as it is possible to specify the operator, who is allowed to see the radiographing information. As another example, the operator identification information may be a password common to a plurality of operators, image data of a handwritten signature of an operator, fingerprint data, voice data or the like may be used, instead.

Moreover, it may be also possible to input a plurality of identification information in order to authenticate an operator with a combination thereof. For example, the operator has to input the operator ID and the fingerprint data, and only when both the identification information corresponds, the operator is authenticated. Thereby, the security function can be further improved.

Moreover, in the above description, the order authentication process is executed in response to instruction of displaying the radiographing order information at the time of start-up of the PDA 210. However, for example, it may be possible to authenticate both login to the PDA 210 and the display of radiographing order information, in conjunction with the start-up of the PDA 210.

Concretely, when power is ON and the start-up of the PDA 210 is instructed, the PDA 210 displays the input screen for inputting operator ID. When the input operator ID is authenticated, the login to the PDA 210 is granted and then the PDA 210 displays a list screen of the radiographing order information on the display unit 213. When the operator ID is not authenticated, authentication error guidance is given, and the login to the PDA 210 and the display of radiographing order information are not granted and therefore not performed. Since the login is not performed also when the operator ID is not authenticated, the security function of the PDA 210 handling the radiographing order information can be improved.

Also, the detailed structure and the detailed operation of the PDA 210 according to the second embodiment can be suitably modified without departing from the essence of the present invention.

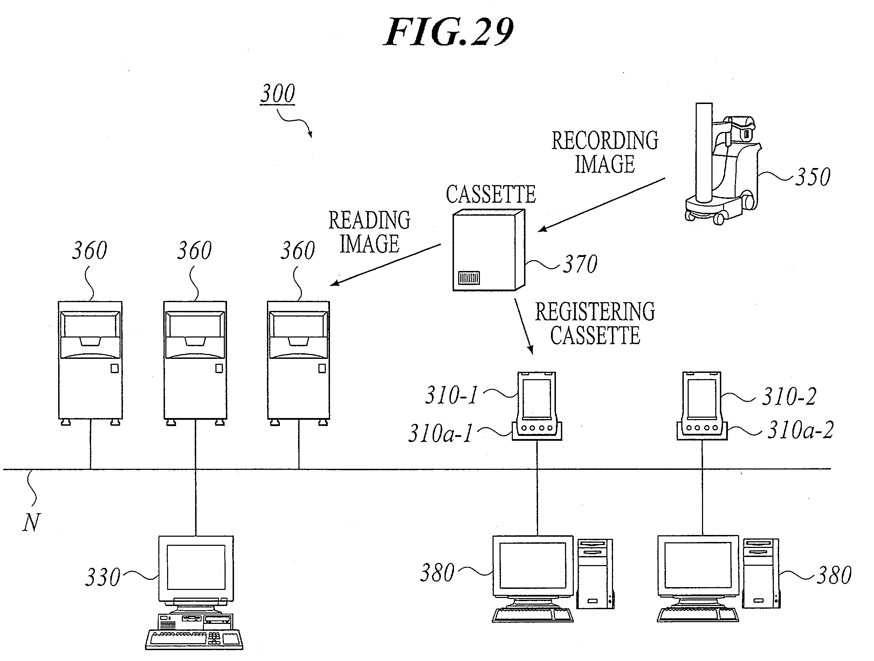

Moreover, the medical image radiographing system 200 may be configured as shown in FIG. 29 as another form.

FIG. 29 is a view showing a system structure of a medical image radiographing system 300, one of modified examples of the medical image radiographing system 200.

In FIG. 29, the medical image radiographing system 300 comprises plurality of PDAs 310, communication terminals 310a corresponding to the plurality of PDAs 310, an information management apparatus 330, a portable radiographing apparatus 350, a medical image reading apparatus 360 for cassette use, a cassette 370 and a control apparatus 380. Each apparatus is connected to a network N and capable of transmitting and receiving data to each other. Functions of the plurality of PDAs 310, the information management apparatus 330, the radiographing apparatus 350, the medical image reading apparatus 360 for cassette use, the cassette 370 and the control apparatus 380 in the medical image radiographing system 300 are respectively approximately the same as the functions of the PDA 210, the information management apparatus 230, the portable radiographing apparatus 250, the medical image reading apparatus 260 for cassette use, the cassette 270 and the control apparatus 280 in the medical image radiographing system 200. Accordingly, the description of the functions is omitted.

Here, after the control apparatus 380 obtains the radiographing order information from the information management apparatus 330, the control apparatus 380 may transmit the radiographing order information only to one assigned by using an IP address among the plurality of PDAs 310, for example, a PDA 310-1, and not transmit it to unassigned one, a PDA 310-2. As a result, the radiographing order information is displayed only on the assigned PDA 310-1. Consequently, the redundant preparation of radiography and redundant radiography can be prevented.

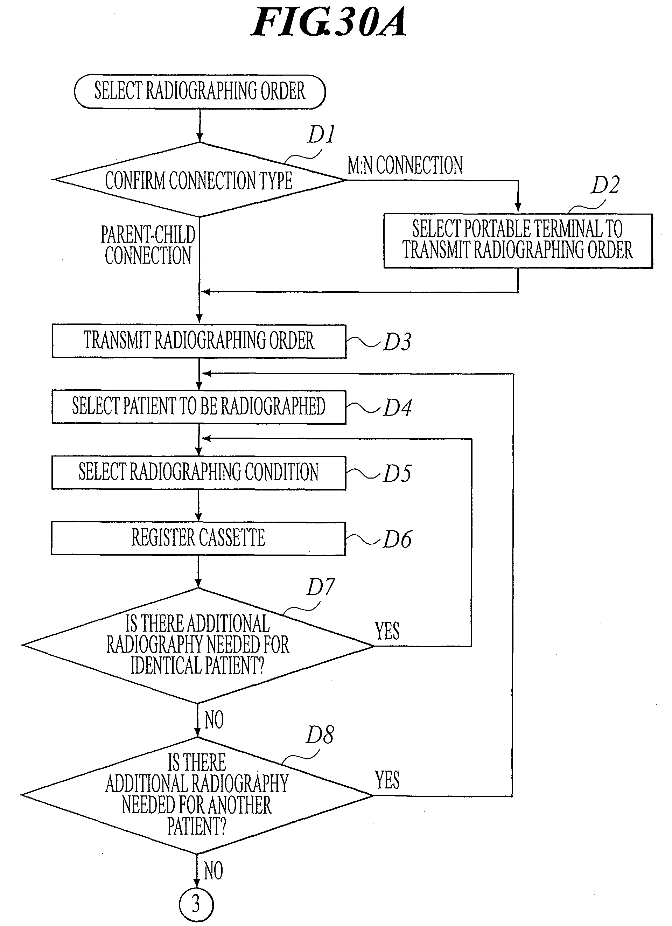

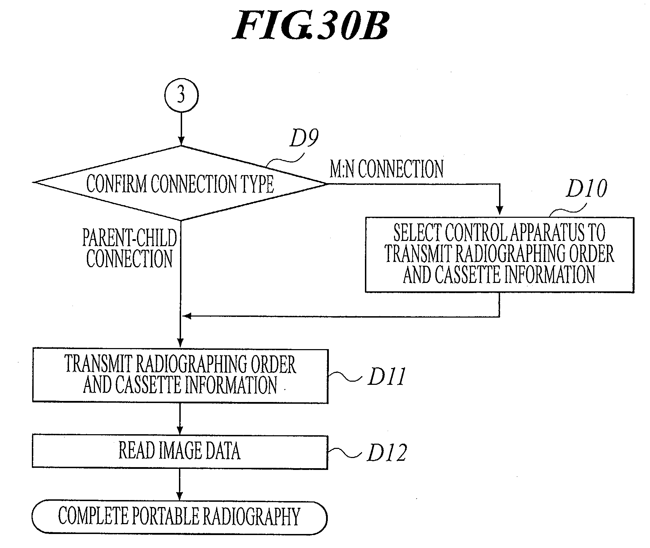

As written above, radiographing processes of, a medical image radiographing apparatus with a connection type of the first embodiment, called a Parent-Child connection, where a portable terminal can only transmit and receive data with a specific control apparatus, and a medical image radiographing apparatus with a connection type of the second embodiment, called an M:N connection, where a portable terminal is directly connected to a network and capable of transmitting and receiving data with any control apparatus are described separately. Hereinafter, a medical image radiographing system capable of performing the radiographing processes appropriate to both the connection types between the portable terminal and the control apparatus, will be described with reference to a flowchart in FIG. 30.

In FIG. 30, when radiographing order information is selected at the control apparatus to be used for radiography, a connection type between the portable terminal and the control apparatus is confirmed (Step D1). In a case that the connection type is the M:N connection (Step D1; M:N CONNECTION), a process to select a portable terminal to transmit the radiographing order information is performed (Step D2), and after the selection, the control apparatus transmits the radiographing order information to the selected portable terminal (Step D3). On the other hand, in a case that the connection type is the Parent-Child connection (Step D1; PARENT-CHILD CONNECTION), the control apparatus transmits the radiographing order information to a predetermined portable terminal (Step D3).

Next, an operator conveys a portable radiographing apparatus and a cassette to a hospital room, and selects a patient to be radiographed from a patient list screen displayed on the portable terminal (Step D4). The portable terminal displays radiographing order information corresponding to the selected patient, and the operator selects a radiographing condition in the radiographing order information (Step D5), and registers the cassette related to the selected radiographing condition (Step D6). The operator performs radiography corresponding to the radiographing order information with the registered cassette. In a case that there is additional radiography needed for the identical patient (Step D7; YES), it is necessary to return to Step D5 and repeat the same processes until all the needed radiography for the identical patient is performed (Step D7; NO). Then, in a case that there is additional radiography needed for other patients (Step D8; YES), it is necessary to return to Step D4 and repeat the same processes until all the needed radiography is performed (Step D8; NO). Here, in the case of the M:N connection (Step D9; M:N CONNECTION), after a process to select a control apparatus to transmit the radiographing order information and cassette information is performed (Step D10), the portable terminal transmits the radiographing order information and the cassette information to the selected control apparatus (Step D11). On the other hand, in the case of the Parent-Child connection (Step D9; PARENT-CHILD CONNECTION), the portable terminal transmits the radiographing order information and the cassette information to a predetermined control apparatus (Step D11).

After the series of radiography are performed, the operator inserts the cassette wherein radiographed image data is recorded into a reading apparatus for reading the image data (Step D12). Meanwhile, the control apparatus relates the read image data with the received radiographing order information and cassette information and display Othem on an image confirmation screen.

The entire disclosure of Japanese Patent Applications Nos. Tokugan 2002-317299 filed on October 31, 2002, Tokugan 2003-084801 filed on March 26 including specifications, claims, drawings and summaries are incorporated herein by reference in their entirety.

## Claims

1. A medical image radiographing system comprising a radiographing apparatus for radiographing a medical image of a patient, an information management apparatus for managing radiographing order information, a control apparatus for obtaining the radiographing order information from the information management apparatus through a communication network, and a portable terminal connectable to the control apparatus,
wherein the control apparatus comprises:
a storage section for storing the radiographing order information obtained from the information management apparatus; and
an assigning section for assigning an operator for performing radiography according to the radiographing order information stored,
the control apparatus transmits the radiographing order information to the portable terminal along with operator identification information of the operator assigned, and
the portable terminal comprises:
a storage section for storing the radiographing order information and the operator identification information transmitted from the control apparatus;
an authentication section for authenticating the operator on the basis of the operator identification information stored; and
a display section for displaying the radiographing order information stored when the authentication section authenticates the operator.

2. The system of claim 1, wherein the portable terminal further comprises a warning section for warning that the operator is not assigned, when the authentication section does not authenticate the operator.

3. The system of any one of claims 1 and 2, further comprising a plurality of control apparatuses,
wherein the portable terminal further comprises an input section for inputting radiography completion information for informing that the radiography according to the radiographing order information stored is completed, and transmits order identification information of the radiographing order information according to which the radiography has completed, to a control apparatus which has transmitted the radiographing order information to the portable terminal among the plurality of control apparatuses on the basis of the radiography completion information input, and
each control apparatus transmits the order identification information of the radiographing order information transmitted from the portable terminal to the other control apparatuses, and when all the plurality of control apparatuses receive the order identification information of the radiographing order information from at least one of any one of the plurality of control apparatuses and the information management apparatus, all the plurality of control apparatuses delete the radiographing order information from the storage section.

4. The system of any one of claims 1 to 3, wherein the portable terminal is connectable to a specific control apparatus among the plurality of control apparatuses.

5. The system of any one of claims 1 to 3, wherein the portable terminal is connectable to any of the plurality of control apparatuses.

6. A method for displaying radiographing order information in a medical image radiographing system comprising a radiographing apparatus for radiographing a medical image of a patient, an information management apparatus for managing radiographing order information, a control apparatus for obtaining the radiographing order information from the information management apparatus through a communication network, and a portable terminal connectable to the control apparatus, the method comprising:
storing the radiographing order information obtained from the information management apparatus in a storage section of the control apparatus;
assigning an operator for performing radiography of the radiographing order information stored;
storing operator identification information of the operator assigned and the radiographing order information in a storage section of the portable terminal;
authenticating the operator on the portable terminal on the basis of the operator identification information stored; and
displaying the radiographing order information for which the operator is assigned on a display section of the portable terminal when the operator is authenticated.

7. The method of claim 6, further comprising: warning that the operator is not assigned when the operator is not authenticated.

8. The method of any one of claims 6 and 7, wherein the medical image radiographing system further comprises a plurality of control apparatuses, the method further comprising:
inputting radiography completion information at the portable terminal for informing that the radiography according to the radiographing order information is completed;
transmitting order identification information of the radiographing order information from a control apparatus which has received the order identification information among the plurality of control apparatuses to the other control apparatuses among the plurality of control apparatuses based on the radiography completion information input, the radiographing order information according to which the radiography has completed; and
deleting the radiographing order information according to which the radiography has completed from storage sections of the plurality of the control apparatuses when all the plurality of control apparatuses receive the order identification information of the radiographing order information according to which the radiography has completed from at least one of any one of the plurality of control apparatuses and the information management apparatus.

9. The method of any one of claims 6 to 8, wherein the portable terminal is connectable to a specific control apparatus among the plurality of control apparatuses.

10. The method of any one of claims 6 to 8, wherein the portable terminal is connectable to any of the plurality of control apparatuses.

11. A portable terminal in a medical image radiographing system, comprising:
a receiving section for receiving radiographing order information including information related to a patient;
a display section for displaying the radiographing order information;
an input section for inputting operator identification information of an operator;
an identification information authentication section for authenticating the operator according to the operator identification information input at the input section; and
a display control section for controlling display of the information related to the patient on the display section on the basis of a result of operator authentication by the identification information authentication section.

12. The terminal of claim 11, further comprising a radiographing order information storage section for storing the radiographing order information received by the reception section,
wherein the display control section controls display on the display section by downloading the information related to the patient from the radiographing order information storage section on the basis of the result of operator authentication by the identification information authentication section.

13. The terminal of claim 11, wherein the display control section controls display on the display section by downloading the information related to the patient by way of a control apparatus from an information management apparatus managing the radiographing order information on the basis of the result of operator authentication by the identification information authentication section.

14. The terminal of any one of claims 11 to 13, wherein the identification information authentication section is set to operate in conjunction with a start-up of the portable terminal.

15. A display control method for displaying information related to a patient, comprising:
receiving radiographing order information including the information related to a patient;
displaying the radiographing order information on a display section;
inputting operator identification information of an operator;
authenticating the operator according to the operator identification information input; and
controlling display of the information related to the patient on the display section on the basis of a result of the operator authentication.

16. The method of claim 15, further comprising storing the radiographing order information received,
wherein the controlling display of the information related to the patient includes controlling display on the display section by downloading the information related to the patient included in the radiographing order information stored on the basis of the result of the operator authentication.

17. The method of claim 15, wherein the controlling of the information related to the patient includes controlling display on the display section by downloading the information related to the patient by way of a control apparatus from an information management apparatus managing the radiographing order information on the basis of the result of the operator authentication.

18. The method of any one of claims 15 to 17, wherein the authenticating the operator according to the identification information is done in conjunction with a start-up of the portable terminal.

19. A program for making a computer execute:
receiving radiographing order information including information related to a patient;
displaying the radiographing order information on a display section;
receiving an input of operator identification information of an operator;
authenticating the operator according to the operator identification information input; and
controlling display of the information related to the patient on the display section on the basis of a result of the operator authentication.

20. A medical image radiographing system comprising:
a control apparatus for storing radiographing order information including information related to a patient as downloadable, and capable of transmitting the radiographing order information to an external device in response to downloading instruction;
a network connected to the control apparatus;
a portable terminal for receiving the radiographing order information through the network and displaying the information related to the patient after authenticating an operator when providing the operator the radiographing order information; and
a radiographing apparatus for performing radiography of the patient in accordance with instruction of the operator.

21. The system of claim 20,
wherein the portable terminal comprises:
a receiving section for receiving the radiographing order information including the information related to the patient;
a radiographing order information storage section for storing the radiographing order information received by the receiving section;
a display section for displaying the radiographing order information;
an input section for inputting operator identification information of an operator;
an identification information authentication section for authenticating the operator according to the operator identification information input at the input section; and
a display control section for controlling display of the information related to the patient on the display section on the basis of a result of operator authentication by the identification information authentication section.

22. The system of claim 21,wherein the display control section of the portable terminal controls display on the display section by downloading the information related to the patient from the radiographing order information storage section on the basis of the result of operator authentication by the identification information authentication section.

23. The system of claim 21, wherein the display control section of the portable terminal controls display on the display section by downloading the information related to the patient by way of the control apparatus from the information management apparatus managing the radiographing order information on the basis of the result of operator authentication by the identification information authentication section.

24. The system of any one of claims 21 to 23, wherein the identification information authentication section of the portable terminal is set to operate in conjunction with a start-up of the portable terminal.

25. A medical image radiographing system comprising:
a control apparatus for managing radiographing order information and a medical image related to the radiographing order information;
at least one portable terminal to which the control apparatus is capable of transmitting the radiographing order;
an assigning section for assigning a portable terminal among the at least one portable terminal to which the control apparatus transmits the radiographing order information when the control apparatus transmits the radiographing order apparatus to the at least one portable terminal; and
a communication control section for transmitting the radiographing order information to the portable terminal assigned.

26. The system of claim 25, further comprising a storage section for storing an address allocated for each of the at least one portable terminal;
wherein the assigning section assigns the portable terminal to which the control apparatus transmits the radiographing order information to with the address; and
the communication control section controls communication of the system with the address.

27. The system of claim 26, wherein the control apparatus comprises the assigning section.

28. The system of claim 25, further comprising a plurality of control apparatuses,
wherein the plurality of control apparatuses are connected to each other through a network.

29. The system of claim 28, wherein the at least one portable terminal is capable of communicating with the plurality of control apparatuses through the network.

30. The system of claim 28, wherein the at least one portable terminal is capable of communicating with a predetermined control apparatus among the plurality of control apparatuses.

31. A medical image radiographing system comprising:
a radiographing order generating apparatus for generating radiographing order information;
a control apparatus for obtaining the radiographing order information and relatedly managing the radiographing order information and at least one of information related to the radiographing order information and a medical image;
at least one portable terminal to which the control apparatus is able to transmit the radiographing order;
an assigning section for assigning a portable terminal to which the control apparatus transmits the radiographing order information among the at least one portable terminal when the control apparatus transmits the radiographing order information to the at least one portable terminal; and
a communication control section for transmitting the radiographing order information to the portable terminal assigned.

32. The system of claim 31, further comprising a storage section for storing an address allocated for each of the at least one portable terminal,
wherein the assigning section assigns the portable terminal to which the control apparatus transmits the radiographing order information with the address, and
the communication control section controls communication of the system with the address.

33. The system of claim 32, wherein the control apparatus comprises the assigning section.

34. The system of claim 31, further comprising a plurality of control apparatuses,
wherein the plurality of control apparatuses are connected to each other through a network.

35. The system of claim 34, wherein the at least one portable terminal is capable of communicating with the plurality of control apparatuses through the network.

36. The system of claim 34, wherein the at least one portable terminal is capable of communicating with a predetermined control apparatus among the plurality of control apparatuses.

37. A medical image radiographing system comprising:
at least one control apparatus for managing radiographing order information and a medical image related to the radiographing order information;
at least one portable terminal comprising at least one of a receiving section for receiving the radiographing order information from the at least one control apparatus, and a transmitting section for transmitting a radiography result corresponding to the radiographing order information;
an assigning section for assigning at least one of a portable terminal among the at least one portable terminal to which the at least one control apparatus transmits the radiographing order information when the at least one control apparatus transmits the radiographing order information to the at least one portable terminal, and a control apparatus among the at least one control apparatus to transmit the radiography result to when the at least one portable terminal transmits the radiography result to the at least one control apparatus;
a communication control section, when the assigning section assigns a portable terminal among the at least one portable terminal to transmit the radiographing order to, for transmitting the radiographing order information to the portable terminal, and, when the assigning section assigns a control apparatus among the at least one control apparatuses to transmit the radiography result to, for transmitting the radiography result to the control apparatus.

38. The system of claim 37, further comprising a storage section for storing an address allocated for each of the at least one portable terminal,
wherein the assigning section assigns a portable terminal among the at least one portable terminal to transmit the radiographing order information to with the address, and
the communication control section controls communication of the medical image radiographing system with the address.

39. The system of claim 37, further comprising a storage section for storing an address allocated for each of the at least one control apparatus,
wherein the assigning section assigns a control apparatus among the at least one control apparatus to transmit the radiographing order information according to which radiography has been completed to with the address, and
the communication control section controls communication of the medical image radiographing system with the address.

40. The system of claim 38,
wherein the storage section stores an address allocated for each of the at least one control apparatus, and
the assigning section assigns a control apparatus among the at least one control apparatus to transmit the radiographing order information according to which radiography has been completed to with the address.

41. The system of claim 38, wherein the at least one control apparatus comprises the assigning section.

42. The system of claim 37, wherein each of the at least one control apparatus and the at least one portable terminal comprises the assigning section and the communication control section.

43. The system of claim 37, further comprising a plurality of control apparatuses,
wherein the plurality of control apparatuses are connected to each other through a network.

44. A medical image radiographing system comprising:
a radiographing apparatus for radiographing a medical image of a patient;
an information management apparatus for managing radiographing order information and transmitting the radiographing order information to outside according to instruction through a communication network;
a plurality of control apparatuses for generating a radiographing order list by receiving the radiographing order information from the information management apparatus through the communication network, transmitting the radiographing order information assigned in the radiographing order list to a portable terminal and relating the radiographing order information with radiography result related information transmitted from the portable terminal; and
at least one portable terminal connectable to at least one of the plurality of control apparatuses for transmitting the radiography result related information corresponding to the radiographing order information received from the at least one of the plurality of control apparatuses to one of the plurality of control apparatuses,
wherein when one of the plurality of control apparatuses relates the radiographing order information with the radiography result related information, the radiographing order information is deleted from the radiographing order list of the other control apparatuses.

45. The system of claim 44,
wherein the radiographing apparatus radiographs a radiation image by using a cassette, and
the radiography result related information is identification information of the cassette used for radiographing the radiation image.

46. A medical image radiographing system comprising:
a radiographing apparatus for radiographing a medical image of a patient,
an information management apparatus for managing radiographing order information and transmitting the radiographing order information to outside according to instruction through a communication network,
a plurality of control apparatuses for generating a radiographing order list by receiving the radiographing order information from the information management apparatus through the communication network, transmitting the radiographing order information assigned in the radiographing order list to a portable terminal and relating the radiographing order information with radiography result related information transmitted from the portable terminal; and
at least one portable terminal connectable to at least one of the plurality of control apparatuses for displaying the radiographing order information received from the at least one of the plurality of control apparatuses;
wherein when any one of the plurality of control apparatuses transmits the radiographing order information to any one of the at least one portable terminal, the radiographing order information is deleted from the radiographing order list of the other control apparatuses.
